(19) Europäisches Patentamt / European Patent Office / Office européen des brevets

(11) **EP 1 886 116 B1**

(12) **FASCICULE DE BREVET EUROPEEN**

(45) Date de publication et mention
de la délivrance du brevet:
**11.07.2018 Bulletin 2018/28**

(21) Numéro de dépôt: **06794409.0**

(22) Date de dépôt: **10.05.2006**

(51) Int Cl.:
*G01N 17/00* (2006.01)    *G01N 25/18* (2006.01)

(86) Numéro de dépôt international:
**PCT/FR2006/050424**

(87) Numéro de publication internationale:
**WO 2007/003801 (11.01.2007 Gazette 2007/02)**

(54) **MÉTHODE ET SYSTÈME POUR LA MESURE ET L'ÉTUDE DE L'ENCRASSEMENT D'UN REACTEUR**

VERFAHREN UND SYSTEM ZUR MESSUNG UND PRÜFUNG DER VERSCHMUTZUNG EINES REAKTORS

METHOD AND SYSTEM FOR MEASURING AND EXAMINING REACTOR FOULING

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **10.05.2005 FR 0551209**

(43) Date de publication de la demande:
**13.02.2008 Bulletin 2008/07**

(73) Titulaire: **INSTITUT NATIONAL DE LA RECHERCHE AGRONOMIQUE (INRA)**
**75341 Paris Cedex 07 (FR)**

(72) Inventeurs:
• **FILLAUDEAU, Luc**
  **F-31520 Romainville Saint Agne (FR)**
• **CARDENAS, René**
  **F-44130 Blain (FR)**

• **DEBREYNE, Pascal**
  **F-59273 Fretin (FR)**
• **KOROLZUCK, Josef**
  **F-35000 Rennes (FR)**

(74) Mandataire: **Jacobacci Coralis Harle**
**32, rue de l'Arcade**
**75008 Paris (FR)**

(56) Documents cités:
**WO-A-00/43762     GB-A- 2 089 512**
**US-A- 4 383 438**

• **JONES A D ET AL: "The use of a heat flux sensor in monitoring fouling" CONFERENCE PROCEEDINGS EUROPEAN COMMISSION, SCIENCE RESEARCH AND DEVELOPMENT, 23 mars 1994 (1994-03-23), pages 230-241, XP002088858**

**Description**

## SOMMAIRE

La présente invention se rapporte au domaine de la mesure de l'encrassement dans un échangeur de chaleur ou un chambreur (maintien en température) ou une conduite constituant un élément dans un procédé en continu, tel qu'une ligne de fabrication d'un produit dans l'industrie agro-alimentaire.

## ART ANTERIEUR

Dans les procédés continus, on a recours à des systèmes de traitement comprenant un ou plusieurs réacteurs en série dans lesquels on réalise les étapes successives de traitement, telles que les opérations de stabilisation, de conservation, de cuisson, de refroidissement et de chauffage. Les réacteurs sont connectés ou mis en communication fluidique, les uns avec les autres, par des moyens appropriés, en général des conduites tubulaires.

Quels que soient le procédé de traitement et le fluide considérés , les parois des réacteurs sont le siège de phénomènes d'encrassement. L'encrassement s'accroît avec la durée d'utilisation, ce qui entraîne au moins une réduction du rendement du procédé, et ce qui conduit même dans certains cas à rendre inefficace l'installation, nécessitant son arrêt pour nettoyage. Notamment, le phénomène d'encrassement réduit drastiquement les performances des réacteurs tel que les échangeurs de chaleur ou les zones de chambrage.

Le phénomène d'encrassement est rencontré au cours de tout type de traitement de fluides, notamment dans le domaine du traitement de l'eau, du traitement des hydrocarbures, ou encore de la fabrication de produits d'intérêt agro-alimentaire, y compris de procédés de fabrication de produits agro-alimentaires incluant une ou plusieurs étapes de cuisson. Seules la cinétique, l'intensité, la structure et la nature des encrassements diffèrent.

L'encrassement est un phénomène local qui varie avec les conditions opératoires et la nature du produit à traiter, le temps de traitement du produit dans un réacteur ou une conduite tubulaire, et de la distribution spatiale.

Un suivi de l'évolution de l'encrassement des réacteurs et/ou des tubulures de communication fluidique est essentiel lors de la mise en oeuvre de procédés en continu de traitement des fluides, afin de permettre la détermination du moment ou l'installation de traitement doit être arrêtée pour nettoyage, afin de restaurer les performances de traitement du fluide considéré.

Pour réaliser un tel suivi, différents types de méthodes ou de capteurs sont envisageables : mesures locale ou globale, évaluation du potentiel encrassant d'un produit, mesures intrusive ou non-intrusive, mesures directes ou indirectes, mesures *in situ* lors du procédé (mesure en ligne), ou post opératoire, c'est-à-dire hors ligne.

Divers systèmes de suivi de l'état d'encrassement de réacteurs, principalement du type échangeur de chaleur, sont déjà connus.

On a décrit des systèmes optiques de mesure de l'encrassement. La demande PCT n° WO 00/60633 décrit un tel système comprenant une source lumineuse qui envoie un faisceau traversant l'axe radial du réacteur, et un capteur localisé en face de la source. la transmission de la lumière est mesurée au cours du temps. La valeur de transmission de la lumière est inversement proportionnelle au niveau d'encrassement du réacteur testé.

On a aussi décrit, pour des réacteurs du type échangeur de chaleur, un système comprenant un maillage de conducteurs électriques répartis sur la surface de la paroi du réacteur dans lesquels on fait passer un courant électrique, le maillage de conducteurs électriques étant relié à un moyen de mesure de la résistance locale des conducteurs. L'évolution de l'état d'encrassement du réacteur est suivie par calcul des variations des caractéristiques électriques du maillage de conducteurs, tels que les valeurs de voltage ou de résistance (Demande PCT n° WO 01/94876).

On a également proposé divers systèmes de suivi de l'encrassement d'un réacteur par mesure de l'évolution des températures en différents points du système.

Par exemple, la demande de brevets aux Etats-Unis n° US 2002/0108911 décrit un système de mesure de l'encrassement d'une installation de traitement de l'eau qui comprend deux capteurs de température, respectivement (i) un premier capteur de température localisé sur le circuit fluidique principal et (ii) un second capteur de température localisé sur une dérivation du circuit fluidique principal, ce second capteur étant régulièrement nettoyé. Le suivi de l'encrassement est réalisé par mesure de la différence de température entre le premier capteur, que l'on laisse s'encrasser avec le temps, et le second capteur, qui est régulièrement nettoyé et n'est donc jamais encrassé.

Dans le brevet aux Etats-Unis n° US 5,590,706, on décrit un système de suivi de l'encrassement d'un réacteur du type échangeur de chaleur qui combine, à la fois à l'entrée et à la sortie du réacteur, un capteur de volume de flux du fluide traité avec un capteur de température. Le niveau d'encrassement de l'échangeur de chaleur est déterminé par calcul différentiel de la combinaison volume de flux/température, respectivement à l'entrée et à la sortie du réacteur.

La demande de brevet européen n° EP 0 155 826 divulgue un système de suivi de l'encrassement d'un réacteur du type échangeur de chaleur, qui est constitué de trois capteurs de température, combinés à un moyen de mesure du débit du fluide traité qui traverse le réacteur. Les deux premiers capteurs de température sont localisés respectivement à l'entrée et à la sortie du réacteur, dans le courant du fluide traité qui circule. Le troisième capteur est localisé sur la partie interne de la surface de l'échangeur, où circule le fluide caloporteur. Ce système permet le calcul du coefficient de transfert de chaleur du réacteur au cours du temps, qui varie avec la progression du niveau d'encrassement.

Le brevet aux Etats-Unis n° US 5,615,733 concerne un système de suivi de l'encrassement d'un réacteur du type échangeur de chaleur, qui est constitué de quatre capteurs de température combinés avec un moyen de mesure du débit du fluide traité, localisé à l'entrée du réacteur. Les deux premiers capteurs sont localisés respectivement à l'entrée et à la sortie du réacteur, dans le courant du fluide traité qui circule. Les troisième et quatrième capteurs sont localisés sur la partie interne de la surface de l'échangeur, respectivement à l'entrée et à la sortie du tube dans lequel circule le fluide caloporteur. Ce système permet de suivre l'évolution de la valeur du coefficient de transfert de chaleur du réacteur au cours du temps, qui varie avec la progression du niveau d'encrassement.

Les systèmes connus de mesure du niveau d'encrassement ci-dessus, en particulier ceux comprenant des capteurs de température, peuvent se révéler satisfaisants. Toutefois, ils présentent généralement l'inconvénient d'être complexes, que ce soit dans leur construction ou dans leur mise en oeuvre.

Ainsi, le système décrit dans la demande de brevets aux Etats-Unis n° US 2002/0108911 impose la conception de circuits fluidiques complexes comprenant des tubulures de dérivation. De plus, de tels systèmes nécessitent de réaliser, une succession d'étapes de désencrassement, régulièrement espacées dans le temps, d'au moins l'un des capteurs au cours de l'utilisation de l'installation.

La demande PCTWO0043762 décrit un procédé et un dispositif pour détecter la formation d'un dépôt de matière contenue dans un fluide sous forme liquide ou gazeuse sur une surface. Le dispositif décrit dans WO0043762 est basé sur l'utilisation d'un capteur de flux thermique dont la première face est en contact avec le fluide et la seconde face peut être en contact avec un élément chauffant de type résistance électrique. Le dispositif comprend également des moyens de mesure de la différence de températures entre le fluide et la deuxième face du capteur. Le procédé selon WO0043762 comprend de mesurer en continu la valeur du flux thermique qui circule entre la première et la deuxième face du capteur tout en maintenant constante la différence de température entre les deux faces du capteur.

La demande de brevet GB 2089512 décrit un dispositif pour mesurer l'encrassement de conduites fluidiques ainsi que différents paramètres du fluide tel que la conductivité, le pH, la turbidité et la corrosion. Le dispositif décrit dans GB 2089512 comprend un assemblage de conduites comportant une entrée et une sortie de fluide ainsi qu'un assemblage d'essai de transfert de chaleur comportant un élément chauffant disposé à l'intérieur d'un conduit dans lequel est ménagé un passage pour ce fluide; un moyen pour mesurer la température du fluide pénétrant dans cet assemblage d'essai de transfert de chaleur; un élément alimentant l'élément chauffant en une quantité présélectionnée d'énergie électrique; un moyen pour mesurer la température des parois de l'élément chauffant; un élément destiné à mesurer la vitesse du fluide à travers la conduite précitée; et un élément fournissant des données relatives à l'encrassement à partir de la quantité présélectionnée d'énergie électrique amenée à cet élément chauffant, de la température du fluide et de la température des parois de l'élément chauffant.

Le brevet aux Etats-Unis n°US 4,383,438 décrit un dispositif permettant de mesurer la capacité d'encrassement d'un fluide en laboratoire. Ce dispositif comprend, entre autres, un réacteur cylindrique pouvant être mis sous pression, un moyen pour contrôler la température du fluide dans le réacteur, une sonde cylindrique ayant une surface métallique, positionnée dans le réacteur et possédant un moyen pour chauffer sa surface, un moyen de mesure de la température à la surface de la sonde et un moyen pour mesurer la température du fluide.

Les divers autres systèmes décrits ci-dessus combinent une multiplicité de capteurs de température à des moyens de mesure du débit du fluide traité, voire aussi des moyens de mesure du débit du fluide caloporteur.

En outre, la plupart des systèmes ci-dessus ne peuvent être utilisés pour le suivi de l'encrassement que d'un seul type de matériel, à savoir des réacteurs du type échangeur de chaleur.

Il existe un besoin dans l'état de la technique pour des systèmes de mesure de l'encrassement de réacteurs, qui soient plus simples à installer et à utiliser que les dispositifs connus.

De plus, il existe un besoin pour des systèmes qui soient adaptés pour le suivi de l'encrassement de n'importe quel organe d'une installation de traitement d'un fluide, à savoir indifféremment d'une tubulure de communication fluidique aussi bien que d'un réacteur, qu'il s'agisse d'un réacteur du type échangeur de chaleur ou de tout autre type de réacteur, y compris un réacteur isotherme communément appelé réacteur de chambrage.

Un tel système permettant le suivi de l'encrassement d'une installation de traitement de fluides est fourni selon l'invention.

## SOMMAIRE DE L'INVENTION

La présente invention se rapporte à une méthode pour la mesure de l'encrassement d'un réacteur (R) dans lequel circule un fluide à température ($\theta_b$), ledit réacteur comprenant un système de mesure de l'encrassement comprenant :

(i) localisée dans le coeur de l'écoulement fluidique du réacteur (R), une sonde active constituée d'un générateur de chaleur (1), cylindrique ou plan, sur la surface externe duquel est fixée une première sonde thermique (2) ; et
(ii) localisée à proximité du générateur de chaleur (1), également dans le coeur de l'écoulement fluidique du réacteur (R), une sonde passive constituée d'une deuxième sonde thermique (3) ;

ledit procédé comprenant les étapes suivantes :

a) porter la surface externe de la paroi dudit générateur de chaleur (1) à la température ($\theta_w$), la température ($\theta_w$) étant mesurée avec la sonde thermique (2), et ($\theta_w$) différant d'au plus de +0,5°C par rapport à la température ($\theta_b$) du fluide en circulation dans le réacteur (R) en l'absence d'encrassement, la température ($\theta_b$) étant mesurée avec la sonde thermique (3) ;
b) mesurer, au cours du temps de fonctionnement du réacteur (R), respectivement les températures ($\theta_w$) et ($\theta_b$) et calculer la valeur de différence ($\Delta\theta = \theta_w - \theta_b$) ; et
c) déterminer l'état d'encrassement du réacteur (R), qui varie avec la valeur de $\Delta\theta$.

L'invention est également relative à un système pour la mesure de l'encrassement d'un réacteur selon la revendication 14.

## DESCRIPTION DES FIGURES

La **Figure 1** illustre le schéma d'un système de mesure de l'encrassement d'un réacteur selon l'invention.
La **Figure 2** illustre un schéma détaillé d'un mode de réalisation particulier d'un système de mesure de l'encrassement d'un réacteur selon l'invention, qui comprend un générateur de chaleur du type sonde thermique à fil chaud et des sondes de température (thermocouple et sonde de platine).
Les **Figures 3** et **4** illustrent les résultats d'un suivi de l'état d'encrassement d'un réacteur de chambrage contenu dans une installation de traitement d'un produit laitier, qui ont été obtenus grâce à la méthode de mesure selon l'invention. Sur les figures 3 et 4, le produit à traiter est introduit dans le réacteur (R) au temps T=0.
La **Figure 5** illustre le domaine ou la plage de travail du générateur de chaleur (1) pour son utilisation dans un dispositif de détection de l'encrassement d'un réacteur (R) selon l'invention.

En ordonnées, à droite : intensité de courant appliquée au générateur de chaleur (1) ; en ordonnées à gauche : puissance générée.
Le domaine ou la plage de travail du générateur de chaleur (1) comme détecteur d'encrassage consiste en la surface médiane de la figure qui est représentée en couleur grise. Les résultats représentés sur la figure ont été obtenus avec un générateur de chaleur (1) consistant en une sonde de platine à fil chaud ayant une résistance de 100 $\Omega$, une longueur L de 30 mm et un diamètre de 1,6 mm. Avec un tel générateur de chaleur (1), le domaine ou la plage de travail du dispositif de détection d'encrassement est de (i) 5mA<Intensité<50 mA et (ii) 2,5 mW<Puissance<250 mW.
Pour une intensité de courant inférieure, le générateur de chaleur (1) se comporte comme une sonde de température, dans le domaine ou la plage représentée en couleur noire, dans la partie inférieure de la figure.
Pour une intensité de courant supérieure, le générateur de chaleur (1) se comporte exclusivement comme une source de chaleur, dans le domaine ou la plage qui est représentée en couleur banche, dans la partie supérieure de la figure.

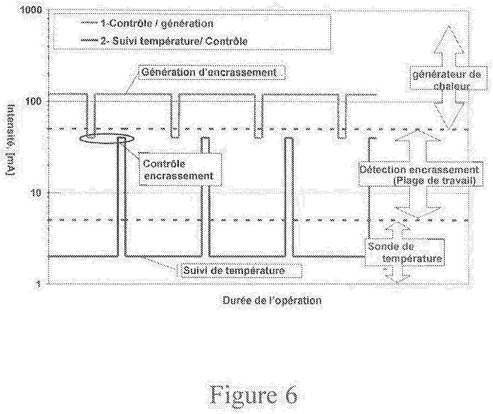

La **Figure 6** illustre deux modes d'utilisation possible du dispositif de détection de l'encrassement selon l'invention à l'étude de l'encrassement d'un réacteur (R).
En ordonnées, l'intensité de courant appliquée au générateur de chaleur (1) ; en abscisse, représentation arbitraire de la durée de étude.
Sur la figure, on représente, par la courbe inférieure de couleur noire, des séquences d'application d'une variation d'intensité dans le générateur de chaleur (1) avec le temps correspondant à une phase de mesure de température (Intensité faible) suivie d'une phase de contrôle de l'encrassement à fréquence fixe.
Sur la figure, on représente, par la courbe supérieure, des séquences d'application d'une variation d'intensité dans le générateur de chaleur (1) avec le temps au cours desquelles alterne à fréquence fixe une phase de génération d'un encrassement (Intensité élevée) suivi d'une phase de contrôle de l'encrassement.

## DESCRIPTION DETAILLEE DE L'INVENTION

On a montré selon l'invention que l'on peut réaliser le suivi de l'encrassement d'un réacteur dans lequel circule un fluide, par une méthode de mesure qui ne nécessite pas le calcul d'un coefficient de transfert de chaleur caractérisant ledit réacteur, ni a fortiori le calcul d'une variation de ce coefficient, comme paramètre caractérisant l'état dudit réacteur.

Au contraire l'invention fournit une méthode de mesure de l'état d'encrassement d'un réacteur qui ne nécessite que la mesure d'une différence de température entre, d'une part, la température du fluide en circulation dans le réacteur et, d'autre part, la température de la paroi externe d'un générateur de chaleur, cette dernière étant contrôlée de manière à être identique, ou quasi-identique, à la température du fluide en circulation, lorsqu'il n'y a pas d'encrassement du réacteur.

La présente invention a pour objet une méthode pour la mesure de l'encrassement d'un réacteur (R) dans lequel circule un fluide à température ($\theta_b$), ledit réacteur comprenant un système de mesure de l'encrassement comprenant :

(i) localisée dans le coeur de l'écoulement fluidique du réacteur (R), une sonde active comprenant un générateur de chaleur (1), cylindrique ou plan, sur la surface externe duquel est fixée une première sonde thermique (2) ; et
(ii) localisée sur la zone de flux thermique définie par le générateur de chaleur (1), également dans le coeur de l'écoulement fluidique du réacteur (R), une sonde passive constituée d'une deuxième sonde thermique (3) ;

ledit procédé comprenant les étapes suivantes :

a) porter la surface externe de la paroi dudit générateur de chaleur (1) à la température ($\theta_w$), la température ($\theta_w$) étant mesurée avec la sonde thermique (2), et ($\theta_w$) différant d'au plus de +0.5 par rapport à la température ($\theta_b$) du fluide en circulation dans le réacteur (R) en l'absence d'encrassement, la température ($\theta_b$) étant mesurée avec la sonde thermique (3) ;
b) mesurer, au cours du temps de fonctionnement du réacteur (R), respectivement les températures ($\theta_w$) et ($\theta_b$) et calculer la valeur de différence ($\Delta\theta = \theta_w - \theta_b$) ; et
c) déterminer l'état d'encrassement du réacteur (R) qui varie avec la valeur de $\Delta\theta$.

Dans le système de mesure de l'encrassement utilisé par la mise en oeuvre du procédé ci-dessus, la première sonde thermique (2) est fixée sur la surface externe du générateur de chaleur (1). Ladite sonde thermique (2) est donc localisée sur la zone de flux thermique du générateur (1) et à l'interface paroi du générateur (1)/« milieu ». Le milieu est le fluide en cours de traitement dans le réacteur (R) lorsqu'on se place en conditions propres et que le réacteur (R) n'est pas encrassé. Le « milieu » consiste en un matériau d'encrassement lorsque le réacteur (R) est encrassé.

Par « réacteur », on entend selon l'invention tout organe d'une installation de traitement d'un fluide dans lequel circule ledit fluide. Ainsi, un « réacteur » comprend les réacteurs proprement dits d'une installation de traitement d'un fluide, selon la terminologie classiquement utilisée par l'homme du métier. Le terme « réacteur » englobe aussi, selon l'invention, toute tubulure d'une installation qui permet l'alimentation de ladite installation en fluide de départ à traiter, toute tubulure qui permet d'évacuer le fluide traiter de ladite installation, et toute tubulure qui met en communication fluidique deux organes distincts de l'installation, par exemple deux réacteurs distincts, au sens conventionnel du terme. Le terme « réacteur » englobe notamment les réacteurs du type échangeur de chaleur, y compris les échangeurs tubulaires, qui permettent la réalisation des étapes de chauffage ou de refroidissement du fluide qui est traité. Tout particulièrement, le terme « réacteur » englobe les réacteurs de tout type distinct d'un réacteur échangeur de chaleur, y compris les réacteurs dits « de chambrage », qui consistent en des réacteurs isothermes, ou quasi-isothermes, qui permettent la réalisation d'une étape isotherme, ou quasi-isotherme, de transformation du fluide en cours de traitement, y compris une étape de transformation par la chaleur, telle que la cuisson du fluide en cours de traitement, y compris une étape de maintien en température de transformation du fluide par la chaleur, par exemple une cuisson du fluide en cours de traitement.

Par « fluide », on entend selon l'invention tout produit liquide et pompable dont la viscosité n'est pas si grande qu'elle empêcherait sa circulation, de l'entrée vers la sortie de l'installation de traitement, à travers chaque réacteur effectuant une étape d'un procédé de traitement de fluides. Par exemple, la méthode de l'invention est applicable à la mesure de l'encrassement de réacteurs utilisés pour des procédés de traitement de fluides tels que de l'eau, des hydrocarbures, des boues d'eau résiduaire, des produits pâteux d'intérêt agro-alimentaire ou pour d'autres industries, tels que les sucres, l'amidon, les purées végétales, les pâtes de base pour la fabrication des aliments pour les humains ou les animaux.

Selon la méthode de mesure de l'encrassement de l'invention, la surface externe de la paroi du générateur de chaleur (1) de la sonde active, qui est en contact direct avec le fluide en circulation dans le réacteur lorsqu'il n'y a pas encrassement, est portée à une température qui est idéalement identique, à la précision de mesure près, à la température du fluide en cours de traitement dans le réacteur. En d'autres termes, lorsqu'il n'y a pas d'encrassement du réacteur, la différence entre la température du fluide en circulation, qui est mesuré par la sonde passive (3), et la température à la surface de la sonde active, est nulle ou quasi-nulle. Pour atteindre cet état, l'énergie qui est fournie au générateur de chaleur (1) est contrôlée de manière à ce que la température de la surface de paroi externe du générateur (1), qui est mesurée avec la sonde thermique (2), soit identique, aux erreurs de mesure ou de contrôle près, à la température mesurée par la sonde passive (3). En d'autres termes, on « applique » une différence de température ($\Delta\theta = \theta_w - \theta_b$) nulle ou quasi-nulle, au plus égale à 0,5°C (précision de mesure de la sonde thermique (2)), entre la surface externe de la paroi du générateur de chaleur (1) et le fluide en circulation (1), dans le réacteur propre non encrassé.

En pratique, une étape d'étalonnage préalable dans le réacteur non encrassé permet de déterminer à l'avance, pour un fluide à traiter donné, quelle quantité d'énergie doit être fournie par le générateur de chaleur (1) pour que la surface de sa paroi externe en contact avec le fluide soit à la température $\theta_w$ identique, ou quasi-identique, à la température $\theta_b$ du fluide en circulation dans le réacteur.

Avantageusement, la différence entre la température $\theta_w$ de la sonde passive et la température $\theta_b$ du fluide en circulation sera inférieure ou égale à 0,5°C, mieux d'au plus 0,4°C, encore mieux d'au plus 0,3°C, préférentiellement d'au plus 0,2°C et de manière tout à fait préférée d'au plus 0,1°C ou même d'au plus 0,05°C.

La différence entre la température $\theta_w$ de la sonde passive et la température $\theta_b$ du fluide en circulation dépend notamment de la précision de mesure de la température par les sondes (2) et (3). Plus les sondes sont précises, plus la différence de température $\Delta\theta$ peut être faible.

Pour mettre en oeuvre la mesure de l'état d'encrassement d'un réacteur (R), il est essentiel que, dans les conditions où le réacteur n'est pas encrassé (« conditions propres »), la température $\theta_w$ soit identique, ou quasi-identique, à la température $\theta_b$ du fluide en circulation dans le réacteur (R), afin que l'encrassement progressif de la paroi externe du générateur de chaleur (1) simule le plus fidèlement possible l'encrassement simultané des parois du réacteur (R). Par exemple, si la paroi externe du générateur de chaleur (1) était portée à une température $\theta_w$ distincte, significativement supérieure ou inférieure, à celle du fluide circulant dans le réacteur (R), la vitesse d'encrassement du générateur de chaleur (1) ne simulerait pas la vitesse d'encrassement simultané du réacteur (R). Il n'y aurait pas correspondance, pour un instant donné, entre l'état d'encrassement du générateur de chaleur (1) et l'état d'encrassement des parois du réacteur (R).

Au cours du temps de mise en oeuvre d'une installation de traitement d'un fluide, divers dépôts se forment sur les parois internes des tubulures et des réacteurs, ce qui, notamment, réduisent leur diamètre interne efficace et font considérablement varier, notamment, leur débit et en conséquence la durée de passage du fluide en cours de traitement à l'intérieur d'un réacteur donné. Avec la méthode de l'invention, les dépôts d'encrassement se forment simultanément sur les parois du réacteur (R) et sur la paroi de la sonde active qui est localisée dans le coeur de l'écoulement fluidique du réacteur.

Selon la méthode de l'invention, il apparaît, au fur et à mesure de l'accroissement de l'épaisseur de la couche de dépôts d'encrassement à la surface de la sonde active, une variation du transfert de chaleur du générateur de chaleur (1) vers le fluide circulant dans le réacteur. Le coefficient global d'échange (K) diminue lorsque l'épaisseur du dépôt encrassant augmente. Pour une épaisseur (e) donnée de la couche de dépôt d'encrassement, la valeur du coefficient global d'échange dépend de la valeur de la conductivité thermique $\lambda_d$ du dépôt encrassant et de la géométrie de la sonde. En d'autres termes, avec l'encrassement du réacteur, y compris de la sonde active, on mesure une différence entre la valeur de température $\theta_w$, qui est mesurée par la sonde thermique (2) de la sonde active, et la valeur de température $\theta_b$ du fluide circulant, qui est mesurée par la deuxième sonde thermique (3), l'unique élément de la sonde passive.

Au démarrage du procédé de traitement d'un fluide, lorsque les parois du réacteur (R) et de la sonde active sont propres, et du fait d'une densité de flux de chaleur modéré (de 0 à 2 kW/m$^2$), d'un régime thermique et hydraulique stationnaire, mais hydrauliquement et thermiquement non établis du fait d'une longueur très faible, (de quelques millimètres) ; le coefficient de transfert de chaleur (h) atteint une valeur élevée, (de quelques 100 à quelques 1000 W.m$^{-2}$.K$^{-1}$). Dans ces conditions, on postule que les couches limites hydraulique et thermique se développent autour de tout objet immergé dans ledit réacteur, y compris autour des sondes active et passive utilisées dans la méthode de mesure selon l'invention. Le faible flux de chaleur qui est dissipé par le générateur de chaleur (1) de la sonde active provoque une différence de température quasi-nulle, entre la surface externe de la paroi du générateur de chaleur (1) et le fluide circulant dans le réacteur (1). La différence de température ($\Delta\theta = \theta_w - \theta_b$), qui est nulle ou quasi-nulle lorsque le réacteur (R) n'est pas encrassé, résulte du flux de chaleur ci-dessus et du coefficient de transfert de chaleur global (K). Avec le flux de chaleur constant qui est généré, le dépôt progressif d'une couche d'encrassement sur les parois du générateur de chaleur (1), qui correspond au dépôt progressif d'une couche d'encrassement de même épaisseur (e) sur les parois du réacteur (R), provoque une variation de la valeur du coefficient de transfert thermique (K), et en conséquence également une variation de la valeur de la différence de température ($\Delta\theta = \theta_w - \theta_b$).

Avec le procédé et le dispositif de l'invention, le demandeur a recherché l'obtention d'une grande sensibilité de détection de l'encrassage du réacteur (R). Ainsi, selon le procédé de l'invention, les températures internes ($\theta_w$) et externe ($\theta_b$) sont contrôlées, et le flux de chaleur est imposé au dispositif. La précision de la mesure de l'encrassement du réacteur (R) est limitée par la précision du contrôle des températures ($\theta_w$) et ($\theta_b$).

Selon le procédé, le seuil de détection d'une variation dans la différence de température ($\theta_w - \theta_b$).est considéré

comme étant la différence de température minimum pour détecter un dépôt d'encrassement significatif dans le réacteur (R).

Dans les conditions expérimentales du procédé de l'invention, on détecte un encrassement à partir d'une épaisseur d'encrassement de 0,5 mm, et même de 0,1mm, pour une puissance de chauffe de 0,2 W et pour des encrassements alimentaires en cours de traitement ayant une conductivité thermique allant de 0,1 à 0,6 $W.m^{-1}.K^{-1}$.

Le dispositif pour la mesure de l'encrassement selon l'invention peut être également utilisé pour l'étude et la compréhension des phénomènes d'encrassage, notamment des vitesses de réaction d'encrassage, par rapport à des conditions témoin de traitement (température du produit à traiter et température de paroi, flux de chaleur, force de cisaillement). Le dispositif pour la mesure d'encrassement selon l'invention peut être également utilisé pour déterminer les propriétés d'un dépôt d'encrassement (conductivité thermique, cinétique d'encrassage, structure et composition du dépôt). Le dispositif de mesure de l'encrassement selon l'invention peut être utilisé pour étudier la corrélation entre la nature de la surface du réacteur (R), par exemple surface traitée ou enduite, les conditions thermiques et hydrauliques, et le mécanisme d'encrassage. Le dispositif de mesure de l'encrassement selon l'invention peut être également utilisé pour créer un dépôt dans des conditions définies, en relation avec la géométrie de l'échangeur de chaleur (condition de travail critique induisant un phénomène d'encrassage). Ainsi, avec le dispositif de mesure de l'encrassage selon l'invention, on peut réaliser une étude de l'encrassement d'une installation à petite échelle, pour un coût réduit. Une illustration d'une utilisation du dispositif de mesure de l'encrassement selon l'invention pour étudier les phénomènes d'encrassement d'un réacteur (R) est illustré sur la Figure 6.

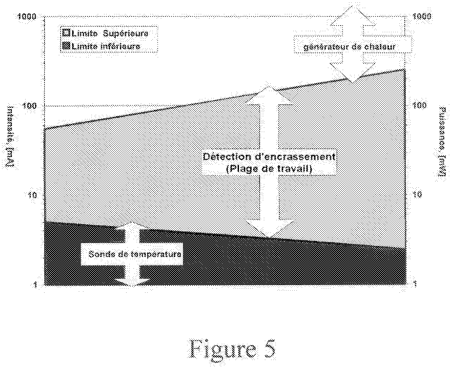

L'utilisation du dispositif de mesure de l'encrassement selon l'invention, comme cela a déjà été mentionné précédemment, requiert le contrôle de l'environnement hydraulique et thermique en relation avec la structure de l'installation et les conditions d'un procédé de traitement d'un fluide, par exemple les conditions d'un procédé de traitement d'un produit alimentaire. L'utilisation du dispositif de mesure de l'encrassement selon l'invention doit être réalisée dans les conditions de travail, qui correspond à la plage de détection d'encrassage qui est représentée sur la figure 5.

Afin d'utiliser le dispositif de mesure de l'encrassement selon l'invention dans les conditions optimales, de manière à réaliser une mesure de l'encrassement la plus précise possible, l'homme du métier peut avantageusement ajuster de manière optimale les paramètres suivants :

- utiliser des sondes de température de très grande précision (seuil de précision inférieur à 0,1°C) afin d'obtenir un seuil de détection de l'encrassement le plus bas possible.
- imposer une différence de température ($\Delta\theta$) proche de 0, lorsque le réacteur (R) est utilisé dans des conditions propres ;
- ajuster la quantité de chaleur dissipée dans le générateur de chaleur (10mW < P < 250 W) afin de prévenir toute surchauffe et toute génération d'encrassage, qui serait due à une température de paroi excessive.

Pour mettre en oeuvre le procédé selon l'invention dans des conditions optimales, l'homme du métier peut agir sur les conditions suivantes :

- utiliser des sondes de température (2) et (3) de grande précision de mesure, afin d'obtenir une grande sensibilité de détection de l'encrassement.

Par exemple, l'homme du métier peut utiliser des sondes de températures (2) et (3) possédant une précision de mesure de $\pm$ 0,1°C, ou même de $\pm$ 0,05°C ;imposer un flux de chaleur au générateur de chaleur (1) permettant d'atteindre une valeur de différence de température ($\theta_w-\theta_b$) proche de zéro en conditions de réacteur (R) propre, c'est-à-dire non encrassé. Par exemple, l'homme du métier peut imposer un flux de chaleur dissipé par le générateur de chaleur (1), tel que la différence de température ($\theta_w-\theta_b$) soit de 0,1°C au moins, ou même encore soit de 0,05°C au moins.

- Ajuster la quantité de chaleur dissipée dans le générateur de chaleur (1) de manière à prévenir une surchauffe susceptible de générer un encrassement artefactuel sur les parois dudit générateur de chaleur (1). Par exemple, selon la température $\theta_b$ du produit en cours de traitement dans le réacteur (R), la quantité de chaleur dissipée (P) dans ledit générateur de chaleur peut varier de 10mW à 250W.

Comme cela est illustré sur la figure 6, un contrôle précis de l'intensité permet d'utiliser le dispositif de mesure de l'encrassage selon l'invention afin (i) de mesurer la température, dans la zone où ce dispositif fonctionne comme sonde de température (ii) contrôler l'encrassement, dans la plage d'intensité dans laquelle le dispositif fonctionne comme détecteur d'encrassement ou encore (iii) pour créer un dépôt sur les parois par contrôle du flux de chaleur et de la température de la paroi, pour les fortes intensités. Le choix d'un mode de fonctionnement et le passage d'une position à une autre, avec une fréquence prédéterminée, est possible afin de contrôler les périodes dans lesquelles le dispositif de mesure de l'encrassement de l'invention fonctionne comme (i) sonde de température, (ii) détecteur d'encrassement ou (iii) générateur d'encrassement.

Ainsi, l'invention a encore pour objet un procédé d'analyse des caractéristiques d'un réacteur (R) vis-à-vis de la formation d'un dépôt d'encrassement, comprenant les étapes suivantes :

a) placer un dispositif de mesure de l'encrassement selon l'invention dans un réacteur (R) à tester non encrassé, dans lequel circule un fluide test ;

b) appliquer, au générateur de chaleur (1) dudit dispositif de mesure, une valeur d'intensité de courant ($I_1$) pour laquelle ledit dispositif fonctionne comme détecteur d'encrassement, et mesurer la valeur de ($\Delta\theta$);

c) appliquer, au générateur de chaleur (1) dudit dispositif de mesure, une valeur d'intensité de courant ($I_2$); soit supérieure à ($I_1$), pour laquelle le générateur (1) provoque un échauffement des parois dudit dispositif ou soit inférieure à ($I_1$), pour laquelle le générateur (1) est utilisé comme sonde de température;

d) appliquer, au générateur de chaleur (1) dudit dispositif de mesure, une valeur d'intensité de courant ($I_1$) pour laquelle ledit dispositif fonctionne comme détecteur d'encrassement, et mesurer la valeur de ($\Delta\theta$);

e) réitérer les étapes c) et d) pendant le nombre de cycles désiré.

A l'étape a) du procédé ci-dessus, le réacteur (R) peut être tout type de réacteur dont on désire tester les propriétés d'encrassement, dont les parois sont de n'importe quel type de matériau, et de tout type de géométrie.

A l'étape a) du procédé, le fluide test consiste en n'importe quel type de fluide dont on désire caractériser la capacité à encrasser le réacteur (R), dans des conditions opératoires simulant l'utilisation réelle du réacteur (R), lorsque ledit réacteur (R) est intégré dans le circuit fluidique d'une installation de traitement complète. Les conditions opératoires qui sont fixées à l'étape a) du procédé englobent les conditions de température, les conditions de pression et les conditions de vitesse d'écoulement du fluide test à l'intérieur du réacteur (R).

A l'étape b) du procédé, la valeur de l'intensité de courant ($I_1$) qui est fixée en fonction du type de générateur de chaleur (1) utilisé, permet audit générateur de chaleur (1) de fonctionner dans la «plage de travail » qui est illustrée notamment sur la figure 5, pour réaliser la mesure de la valeur de ($\Delta\theta$), qui est normalement presque nulle puisque le réacteur (R) n'est pas encrassé.

A l'étape c) du procédé, la valeur de courant ($I_2$) qui est fixée permet audit générateur de chaleur (1) de fonctionner soit en tant que source de chaleur qui provoque une élévation de la température de paroi ($\theta_w$) dudit générateur susceptible de provoquer un encrassement de ladite paroi; soit en tant que sonde de température .

A l'étape d) du procédé, on ramène la valeur de courant dans le générateur de chaleur (1) à la valeur ($I_1$), et on mesure la nouvelle valeur de ($\Delta\theta$). A l'étape d), on peut comparer la valeur de ($\Delta\theta$) calculée à l'étape a) avec la valeur de ($\Delta\theta$) mesurée au début de l'étape d).

Ainsi, dans certains modes de réalisation du procédé ci-dessus, l'étape d) comprend les sous-étapes suivantes :

d1) appliquer, au générateur de chaleur (1) dudit dispositif de mesure, une valeur d'intensité de courant ($I_1$) pour laquelle ledit dispositif fonctionne comme détecteur d'encrassement, et mesurer la valeur de ($\Delta\theta$) ; et

d2) comparer la valeur de ($\Delta\theta$) calculée à l'étape a) avec la valeur de ($\Delta\theta$) mesurée à l'étape d1).

Selon le procédé ci-dessus, un encrassement du réacteur (R), en réalité du dispositif de mesure de l'invention placé dans ledit réacteur (R), est détecté si on observe une différence entre la valeur de ($\Delta\theta$) calculée à l'étape a) et la valeur de entre la valeur de ($\Delta\theta$) calculée à la fin de l'étape d), c'est à dire à l'étape d1).

On peut réaliser le cycle comprenant la succession d'une étape c) puis d'une étape d) le nombre de fois désiré. Plus le nombre de ces cycles est grand, plus le procédé rend possible une analyse des caractéristiques d'encrassement du réacteur (R) en simulant une utilisation de l'installation complète pendant une longue période de temps. Par exemple, le nombre de cycles peut varier de 2 à 1000, en général de 10 à 200.

Les étapes b), c) et d) peuvent avoir des durées variables, selon le type de réacteur et selon le type de fluide que l'on désire tester. Les étapes b) et d) peuvent être réalisée pendant une longue période de temps, notamment pendant 30 min à 100 h, puisque ce sont les étapes au cours desquelles on est susceptible de générer un dépôt d'encrassement. En général, l'étape c) peut être de courte durée, puisqu'il s'agit d'une étape de mesure. Toutefois, dans tous les cas, l'étape c) est poursuivie jusqu'à ce que la valeur de ($\Delta\theta$) soit stable.

Préférentiellement, la géométrie et les dimensions de la sonde active, c'est à dire essentiellement la géométrie et les dimensions du générateur de chaleur (1), sont choisies de manière à réduire au maximum les effets de bord concernant la dissipation de chaleur, afin d'obtenir une densité de flux de chaleur homogène et contrôlée sur toute la surface du générateur de chaleur (1), et plus particulièrement sur la surface du générateur de chaleur (1) avec laquelle la première sonde thermique (2) est en contact, et afin de s'assurer que la sonde active et la sonde passive sont localisées au coeur de l'écoulement dans le réacteur (R).

A titre illustratif, pour une sonde active ayant une surface plane, comprenant un générateur de chaleur (1) plan, on utilise de préférence un générateur de chaleur pour lequel la longueur (L) et la largeur (l) externes sont d'une dimension supérieure à son épaisseur (E), avec en particulier un rapport (L,l)/E supérieur ou égal à 5.

A titre illustratif, pour une sonde active cylindrique, comprenant un générateur de chaleur (1) cylindrique, on utilise de préférence un générateur de chaleur pour lequel la longueur (L) externe est d'une dimension supérieure à son rayon (r), en particulier un rapport L/r supérieur ou égal à 5. Dans tous les cas, la longueur (L) de la sonde active, et donc du générateur de chaleur (1) est inférieure à la longueur d'établissement d'un régime hydraulique établi (Lhyd) dans le cas d'un régime d'écoulement laminaire dans le réacteur (R)..

$$\frac{\text{Lhyd}}{\text{d}} = \alpha . \text{Re}^{\beta} \quad (\alpha, \beta \in \text{IR}^*)$$

L'homme de l'art se référera avantageusement, pour le calcul des dimensions appropriées du générateur de chaleur, aux ouvrages spécialisés (par exemple : Joulié R., Mécanique des Fluides appliqués, Ellipses Ed., ISBN 2-7298-6768-6 1998 et Gnideski V., Churchill S.W., Single Phase convective heat transfer, dans Heat Exchanger Design Handbook, Hemisphere publishing corporation Ed. , ISBN O - 89116 - 125 - 2, 1983)

La densité de flux ($\varphi$,W/m$^2$) produite par le générateur de chaleur (1) est le rapport entre la puissance (q, [W]) dissipée par ledit générateur et la valeur de surface d'échange (S, [m$^2$]) de sa paroi externe. Dans le mode de réalisation particulier, dans lequel le générateur de chaleur consiste en une sonde thermique à fil chaud, par exemple une sonde thermique à fil de platine, la densité de flux de chaleur dissipée par le générateur (1) suit la formule (A) suivante :

$$\varphi = \frac{q}{S} = \frac{R_{Pt100} \cdot I^2}{S} = K \cdot \left(\theta_{wall} - \theta_{bulk}\right) \qquad \text{(A)},$$

dans laquelle :

- $\varphi$ est la densité de flux de chaleur [W.m$^2$] ;
- q est la quantité de chaleur dissipée [W], qui peut être calculée par le produit de la valeur de différence de potentiel (U, [V]) et de l'intensité de courant (I, [A]) appliqués au générateur (1) ;
- $R_{Pt100}$ [$\Omega$] est la résistance du fil chaud du générateur (1) ;
- I [A] est l'intensité du courant appliqué au générateur (1) ;
- K est le coefficient global d'échange [W.m$^{-2}$.K$^{-1}$] ;
- $\theta_w$ ou $\theta_{wall}$ est la température de la surface externe de la paroi du générateur (1) ;
- $\theta_b$ ou $\theta_{bulk}$ est la température du fluide circulant dans le réacteur (R).

La valeur d'épaisseur (e) de la couche d'encrassement de la sonde active peut être calculée, à partir de la différence de température ($\Delta\theta = \theta_w - \theta_b$), lorsque l'on connaît par ailleurs aussi le coefficient de conductivité thermique ($\lambda_d$) du matériau qui se dépose sur les parois du réacteur (R) en cours de traitement.

D'autres caractéristiques techniques de la méthode de mesure de l'état d'encrassement d'un réacteur (R) sont décrites ci-dessous, notamment en relation avec la description des caractéristiques des divers moyens mis en oeuvre pour la réaliser, y compris la sonde active et la sonde passive.

Comme déjà mentionné, la sonde active est constituée d'un générateur de chaleur (1), qui peut être cylindrique ou plan, sur la paroi externe duquel est fixée une première sonde thermique (2).

Tout type de générateur de chaleur connu peut être utilisé, à condition que la température de la surface de la paroi externe de celui-ci, qui est en contact avec le fluide circulant dans le réacteur (R), puisse être contrôlée avec suffisamment de précision.

Par exemple, on peut utiliser un générateur de chaleur consistant en un dispositif échangeur de chaleur dans lequel circule un fluide caloporteur, qui peut être de tout type connu.

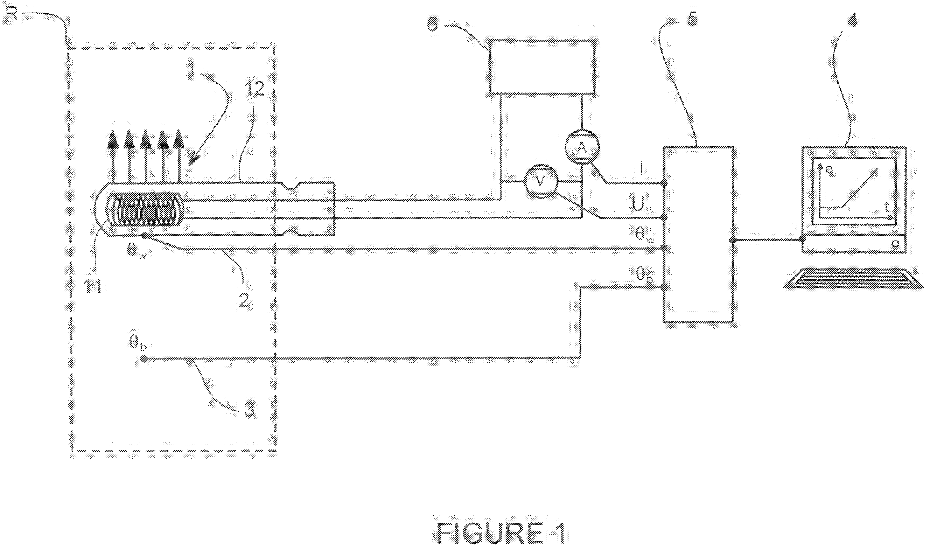

Avantageusement, le générateur de chaleur (1) consiste en un générateur de chaleur comprenant un matériau électriquement conducteur se comportant comme une résistance électrique. En référence à la figure 1, le matériau électriquement conducteur se comportant comme une résistance électrique (11) est isolé de l'environnement du réacteur (R), dans le générateur de chaleur (1), par un étui (12) étanche aux fluides. Le matériau formant l'étui est choisi parmi les matériaux thermiquement conducteurs, tel qu'une résine ou une céramique, ou encore un métal.

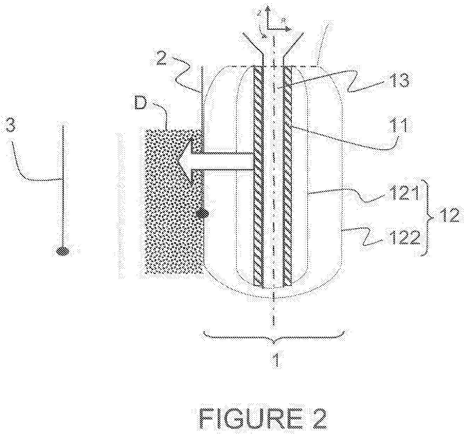

Préférentiellement, le générateur de chaleur consiste en une sonde thermique à fil chaud d'un type connu, telle qu'une sonde thermique à fil de platine, par exemple du type du générateur de chaleur (1) décrit dan la figure 2.

Pour la mise en oeuvre de la méthode de l'invention en utilisant une sonde thermique à fil chaud comme générateur de chaleur, on applique une intensité (I) de courant électrique au fil électriquement conducteur qui est telle qu'elle provoque une génération de chaleur. La densité de flux imposée et contrôlée permet dans les conditions de mesures définies d'obtenir une température de la surface de la paroi externe de la sonde active (2) à une valeur identique ou quasi-identique, à la valeur de température du fluide, en condition propre, et tout en ayant une sensibilité à la formation d'un encrassement éventuel.

Par opposition (1), si une intensité plus faible est imposée, la même sonde à fil chaud fonctionne pour mesurer la température du milieu dans lequel elle est plongée.

Par opposition (2), si une intensité élevée est imposée, la même sonde à fil chaud fonctionne comme source de chaleur et impose des densités de flux élevées. Dans ce cas, la température de surface de la sonde active ne peut être que difficilement identique ou quasi-identique à la valeur de la température du fluide en condition propre (absence d'encrassement).

A titre illustratif, lorsqu'on utilise une sonde thermique à fil chaud du type sonde thermique à fil de platine gainée inox (L = 125mm, L active = 30mm, Ø = 1.6mm, référencée: D52-1716-X-Cu2-125-MMCU commercialisée par la société CORAM (France)), on applique préférentiellement au fil de platine un courant d'une valeur d'intensité (I) qui peut varier de 10 mA à 100 mA, selon la valeur de température de la paroi externe de la sonde qui doit être atteinte. Avec un courant plus faible, par exemple inférieur à 5mA, la même sonde à fil chaud fonctionne pour l'utilisation qui a été prévue initialement par son fabricant, à savoir comme sonde de température.

La sonde thermique à fil chaud illustrée à la figure 2 comprend un fil de platine (11) enroulé autour d'un cylindre en céramique (13) formant l'axe longitudinal de la sonde. L'ensemble constitué du cylindre central de céramique (13) et du fil de platine (11) est enfermé dans un étui (12) étanche aux fluides. L'étui (12) est constitué, de l'intérieur vers l'extérieur de la sonde, d'un matériau électriquement isolant, par exemple en résine ou en céramique, le matériau électriquement isolant étant recouvert d'un film métallique (122), par exemple de l'acier inoxydable, dont la surface externe est en contact avec l'environnement extérieur. De préférence, la paroi externe du générateur de chaleur (1) est résistante à des fluides chimiquement délétères, tels que des fluides extrêmement basiques, comme des solutions de soude, ou extrêmement acides, comme l'acide chlorhydrique ou sulfurique. Par exemple, dans un mode de réalisation particulier, le matériau de la paroi externe du générateur de chaleur (1) est identique au matériau constituant la paroi du réacteur (R).

De manière générale, le générateur de chaleur (1) dissipe une quantité réduite de chaleur par unité de temps et par unité de surface. La quantité de chaleur dissipée par le générateur de chaleur (1) est constante dans le temps. Par exemple, lorsqu'on utilise une sonde thermique à fil chaud comme générateur de chaleur, la source de chaleur est fournie par la résistance électrique, par exemple un fil de platine, par effet Joule direct, lorsqu'on applique à la sonde thermique un courant de valeur constante, variant généralement de 0 mA à 100 mA. Pour une telle sonde thermique, la puissance dissipée constante (q) dépend de la valeur de différence de potentiel (U) et de la valeur de courant (I) appliquée au fil de platine, selon la formule (q=U.I). Dans ces conditions, on évite ainsi une surchauffe de la paroi du générateur de chaleur (1).

Dans la mise en oeuvre de la méthode de l'invention, un moyen de contrôle applique au générateur de chaleur (1) les paramètres appropriés, le cas échéant prédéterminés, qui permettent d'imposer la température de la surface externe de la paroi dudit échangeur à une valeur identique, ou quasi-identique, à la température du fluide circulant dans le réacteur (R), lorsque le réacteur (R) n'est pas encrassé (condition propre).

En référence aux figures 1 et 2, le générateur de chaleur (1) comprend, en contact direct avec la surface externe de la paroi de l'étui (12), une sonde thermique (2).

La sonde thermique (2) peut être tout type de sonde thermique adaptée permettant de mesurer la température de la surface externe de la paroi de l'étui (12) du générateur de chaleur (1). La sonde thermique (2) doit présenter une faible inertie thermique (temps de réponse inférieur à 30 secondes), un faible encombrement géométrique et une précision de mesure satisfaisante.

Préférentiellement, la sonde thermique (2) est une sonde à thermocouple d'un type connu, adaptée à une mesure dans le gamme des températures prévues du fluide circulant dans le réacteur (R).

A titre illustratif, on peut utiliser un thermocouple choisi parmi les types K (Nickel-Chrome/Nickel-Aluminium), T (Cuivre/Cuivre-Nickel), J (Fer/Cuivre-Nickel), N (Nickel-Chrome-Silicium/Nickel-Silicium), E (Nickel-Chrome/Cuivre-Nickel), R (Platine-13% Rhodium/Platine), S (Platine-10% Rhodium/Platine), B (Platine-30% Rhodium/Platine-6% Rhodium), G (Tungstène/Tungsène-26% Rhénium), C (Tungstène-5% Rhenium/Tungstène-26% Rhénium) ou D (Tungstène-3% Rhenium/Tungstène-25% Rhénium).

En particulier, un thermocouple de type K s'avère parfaitement adapté à l'utilisation comme sonde thermique (2).

Préférentiellement, la sonde thermique (2), en particulier lorsqu'il s'agit d'une sonde à thermocouple, est disposée en contact direct avec la surface externe de l'échangeur de chaleur (1), selon l'axe longitudinal de ce dernier. De manière tout à fait préférée, la sonde thermique (2) est contact avec la surface externe de l'échangeur de chaleur (1) sur une longueur allant de 0,25.L à 0,75.L, et mieux sur une longueur allant de 0,3.L à 0,7.L, L étant la longueur de l'échangeur de chaleur (1).

Pour la mise en oeuvre de la méthode de l'invention, la sonde thermique (2) fournit un signal de mesure de la température de la surface externe du générateur de chaleur (1) à un moyen (4) de traitement du signal. Lorsque la sonde thermique (2) est un thermocouple, le signal consiste en une valeur d'intensité du courant appliqué à la sonde, la valeur d'intensité variant avec la valeur de la résistance du thermocouple, laquelle évolue avec la température.

La combinaison du générateur de chaleur (1) et de la sonde thermique (2) sont les éléments essentiels de la sonde active utilisée dans la méthode de mesure de l'encrassement d'un réacteur (R) de l'invention.

Comme déjà décrit précédemment, la méthode selon l'invention utilise aussi une sonde passive qui est constituée d'une deuxième sonde thermique (3). La deuxième sonde thermique (3) est localisée, dans le réacteur (R), à proximité de la sonde active.

La deuxième sonde thermique (3) peut être tout type connu de sonde thermique, qui est adaptée à la mesure de la température du fluide circulant dans le réacteur (R).

La deuxième sonde thermique (3) peut être une sonde thermique à thermocouple, ou encore un autre type de sonde thermique, telle qu'une sonde de Platine dites « Pt 100 »

Selon un autre mode de réalisation, la deuxième sonde thermique (3) est une sonde thermique du type sonde thermique à fil chaud, par exemple une sonde thermique du même type que le dispositif utilisé comme générateur de chaleur (1). Dans le mode de réalisation de la méthode dans lequel la deuxième sonde thermique (3) consiste en une sonde thermique à fil chaud, il est appliqué à ladite sonde thermique une valeur d'intensité adaptée à son utilisation conventionnelle comme sonde thermique, c'est à dire de préférence une intensité de courant inférieure à 10 mA, la valeur d'intensité de courant étant fixée conformément aux recommandations du fabricant.

La deuxième sonde thermique (3) fournit un signal au moyen (4) de traitement du signal.

Le moyen (4) de traitement du signal comprend un moyen de comparaison de la valeur de température $(\theta_w)$ fournie par la première sonde thermique (2) et de la valeur de température $(\theta_b)$ fournie par la deuxième sonde thermique (3), à un instant donné, ledit moyen (4) fournissant, pour cet instant donné, la valeur de la différence $(\Delta\theta = \theta_w - \theta_b)$.

Selon la méthode de mesure de l'invention, on réalise le calcul de la valeur $\Delta\theta$ pour une succession d'instants de mesure simultanée des valeurs de $(\theta_w)$ et de $(\theta_b)$. Par exemple, on peut réaliser la méthode de l'invention en mesurant simultanément $(\theta_w)$ et $(\theta_b)$ pour une succession d'instants donnés, par exemple à des intervalles de temps prédéterminés, puis en calculant la valeur $\Delta\theta$ pour chaque instant successif de mesure. On suit ainsi la progression de la valeur $\Delta\theta$ avec le temps. Les intervalles de temps sont prédéterminés selon le type de procédé de traitement des fluides qui est mis en oeuvre, c'est à dire la vitesse d'encrassement escomptée du réacteur (R) dans lequel on effectue la méthode de mesure de l'invention. Les intervalles de temps sont aussi prédéterminés en fonction du degré de précision que l'on souhaite, concernant l'évolution de l'état d'encrassement du réacteur (R). Ainsi, selon les cas, lesdits intervalles de temps peuvent varier de quelques fractions de secondes à quelques minutes.

Selon la méthode de l'invention, on peut fixer, de manière prédéterminée une valeur limite $\Delta\theta_{Lim}$ à partir de laquelle on considère que le réacteur (R) est dans un état d'encrassement qui justifie d'interrompre son utilisation et de procéder à son nettoyage avant sa prochaine remise en service. La valeur limite $\Delta\theta_{Lim}$ peut être déterminée de manière empirique, par exemple en ayant antérieurement déterminé à partir de quelle valeur de $\Delta\theta$ on observe réellement un encrassement maximum acceptable, au-delà duquel le procédé de traitement du fluide considéré ne peut plus être poursuivi sans affecter son rendement ou son efficacité.

Pour simuler le mieux possible l'encrassement qui est subi par le réacteur (R), la sonde active, qui comprend le générateur de chaleur (1) et la sonde thermique (2), est disposée dans le coeur de l'écoulement fluidique. De préférence, la sonde active est disposée de telle manière que son axe longitudinal soit parallèle à l'axe longitudinal du réacteur (R). Selon une telle orientation, la sonde active est disposée dans le réacteur (R) de manière à ce qu'elle provoque une force de traînée et une résistance à l'écoulement la plus réduite possible, et que la surface de la sonde active exposée au fluide circulant, dans le sens de circulation dudit fluide, soit maximale.

Afin de permettre une mesure précise de la valeur $\Delta\theta$, les extrémités amont, par rapport au sens de l'écoulement fluidique dans le réacteur (R), de la sonde active et de la sonde passive sont de préférence localisées, l'une par rapport à l'autre, dans le sens axial du réacteur (R), à une distance (l) d'au plus 20 cm, mieux d'au plus 10 cm, et encore mieux d'au plus 5 cm.

Afin de permettre une mesure précise de la valeur $\Delta\theta$, la sonde active et la sonde passive sont de préférence localisées, l'une par rapport à l'autre, dans le sens radial du réacteur (R), à une distance (r) d'au plus 0,5 . R, R étant le rayon du réacteur (R).

Préférentiellement, la sonde active et la sonde passive sont localisées sur la même section transversale du réacteur (R).

La méthode de mesure de l'état d'encrassement d'un réacteur selon l'invention est mise en oeuvre, de manière optimale, dans des conditions thermiques et hydrauliques précises dans le réacteur (R) et autour de la sonde active (2).

En régime d'écoulement laminaire, la sonde fonctionne de manière optimale lorsque les variables thermique et hydraulique sont stationnaires, et les régimes thermiques et hydrauliques sont non-établis autour de la sonde active. Cette condition assure un coefficient de transfert thermique par convection optimal et permet d'atteindre $\Delta\theta = (\theta_w - \theta_b)$. $\rightarrow O^+$ en condition propre.

Dans tous les cas, la longueur (L) de la sonde active, et donc du générateur de chaleur (1) est inférieure à la longueur d'établissement d'un régime hydraulique établi (Lhyd) dans le cas d'un régime d'écoulement laminaire dans le réacteur (R).

$$\frac{Lhyd}{d} = \alpha . Re^{\beta} \quad (\alpha, \beta \in \mathbb{IR}^*)$$

L'homme de l'art se référera avantageusement, pour le calcul des dimensions appropriées du générateur de chaleur, aux ouvrages spécialisés (par exemple : Joulié R., Mécanique des Fluides appliqués, Ellipses Ed., ISBN 2-7298-6768-6 1998 et Gnideski V., Churchill S.W., Single Phase convective heat transfer, dans Heat Exchanger Design Handbook, Hemisphere publishing corporation Ed. , ISBN O - 89116 - 125 - 2, 1983)

Comme cela a déjà été mentionné précédemment, le générateur de chaleur (1) peut être plan ou cylindrique.

Selon une caractéristique supplémentaire de la méthode de mesure de l'invention, l'état d'encrassement du réacteur (R) peut aussi être déterminé par un calcul de l'épaisseur (e) de la couche d'encrassement qui s'est déposée à la surface externe de la paroi de la sonde active.

Selon que la géométrie du générateur de chaleur (1), et donc aussi la géométrie de la sonde active, est cylindrique ou plane, le calcul de l'épaisseur (e) de la couche d'encrassement est différent, comme cela est détaillé ci-dessous. Toutefois, dans ces deux modes de réalisation particuliers de la sonde active, le calcul de l'épaisseur (e) de la couche d'encrassement nécessite la connaissance préalable de la valeur du coefficient de conductivité thermique ($\lambda_d$) du matériau constituant la couche d'encrassement.

Dans un procédé de traitement donné d'un fluide spécifique, la composition du matériau qui se dépose sur les parois du réacteur (R) est toujours la même, pour les mises en oeuvre successives dudit procédé de traitement d'un fluide. En conséquence, la valeur du coefficient de conductivité thermique ($\lambda_d$) peut être déterminée au préalable, en mesurant ce paramètre à partir d'un échantillon du matériau d'encrassement prélevé sur les parois du réacteur (R) encrassé. La valeur du paramètre ($\lambda_d$) peut être déterminée selon toute technique conventionnelle de mesure du coefficient thermique d'un matériau solide connue par l'homme du métier. A titre illustratif, le coefficient de conductivité thermique ($\lambda_d$) peut être prédéterminé grâce à un conductimètre thermique.

Dans le mode de réalisation de la sonde active dans lequel le générateur de chaleur (1) est cylindrique, on applique, pour le calcul de l'épaisseur (e) de la couche d'encrassement, la formule (Eq.3) suivante résultant des équations (Eq.1) et (Eq.2).suivantes :

$$P = K \cdot 2 \cdot \pi \cdot r \cdot L \cdot (\theta_w - \theta_b) \quad \textbf{(Eq.1)}$$

with

$$\frac{1}{K} = r \cdot \left( \frac{1}{h \cdot (r + e)} + \frac{Ln(1 + e)}{\lambda_d} \right) \textbf{(Eq.2)}$$

soit :

$$\boxed{\Delta\theta = \theta_w - \theta_b = \frac{P}{2 \cdot \pi \cdot L} \cdot \left( \frac{1}{h \cdot (r + e)} + \frac{1}{\lambda_d} \cdot Ln\left(1 + \frac{e}{r}\right) \right)} \quad \textbf{(Eq.3)}$$

dans laquelle :

- P est la puissance thermique dissipée par le générateur de chaleur (1), la puissance P étant elle-même calculée à partir des grandeurs électriques selon la formule (Eq.4) suivante :

$$P = U.I. \quad \textbf{(Eq.4)}$$

dans laquelle : K est le coefficient global d'échange [$W.m^{-2}.K^{-1}$] ; r est le rayon du générateur de chaleur [m] ; $\theta_w$

est la température de la surface externe de la paroi du générateur de chaleur (1) [K] ; $\theta_b$ est la température du fluide circulant dans le réacteur (R) [K] ; L est la longueur externe du générateur de chaleur (1) [m] ; h est le coefficient de transfert de chaleur par convection [W.m$^{-2}$.K$^{-1}$] ; r est le rayon du générateur de chaleur (1) [m]; e est l'épaisseur de la couche d'encrassement [m] ; et $\lambda_d$ est la valeur du coefficient de conductivité thermique du matériau de la couche d'encrassement [W.m$^{-1}$.K$^{-1}$] ;

Le coefficient de transfert de chaleur par convection, h est régi par les conditions d'écoulement autour de la sonde thermique, et plus spécifiquement autour du générateur de chaleur (1).

L'écoulement se caractérisera, soit par une couche limite turbulente, soit par une couche limite laminaire avec une densité de flux de chaleur uniforme à la surface du générateur de chaleur (1).

L'homme de l'art retrouvera dans la littérature spécifique (Heat Exchange Design Handbook, Hemisphere Publishing Corporation, 1983) l'ensemble des corrélations semi-empiriques permettant d'estimer le coefficient de transfert par convection (h). En général, ces corrélations se formulent comme suit :

Nu = nombre de Nusselt,

$$Nu = \frac{h.L}{\lambda_f}$$

avec $\lambda_f$ : conductivité thermique du fluide [W.m$^{-1}$.K$^{-1}$]

- Re = nombre de Reynolds,

$$Re = \rho \cdot \frac{.U.L}{\mu}$$

avec:

$\rho_f$ : masse volumique du fluide [kg.m$^{-3}$],
U : vitesse d'écoulement du fluide [m.s$^{-1}$], et
$\mu$ : viscosité dynamique du fluide [Pa.s]

$$Pr = \pi \cdot \frac{Cp_f}{\lambda_f}$$

- Pr = nombre de Prandtl     avec $Cp_f$ : chaleur spécifique du fluide [J.kg$^{-1}$.K$^{-1}$]

En pratique, le terme convectif (1/h.(r+e) doit être faible devant le terme de déposition. Ce point est vérifié grâce à une différence de température $\Delta\theta = (\theta_w - \theta_b)$, en condition propre, proche de 0$^+$.

Lors de l'encrassement, le terme de déposition prédomine et l'équation (Eq.3)se simplifie en négligeant le terme convectif :

$$e \cong r.\left( Exp\left( \Delta\theta \cdot \frac{2.L}{P} \cdot \lambda_d \right) - 1 \right) \ (Eq.5)$$

e

avec $\Delta\theta = (\theta_w - \theta_b)$

- $\Delta\theta$ est la différence des températures $(\theta_w - \theta_b)$ [°K] ;
- L est la longueur du générateur de chaleur (1) [m] ;
- h est la valeur de transfert de chaleur par convection [W.m$^{-2}$.K$^{-1}$],
- P est la puissance dissipée dans le fil chaud [W] ;

dans laquelle

- r est le rayon du générateur de chaleur (1) [m] ;

- $\lambda_d$ est la valeur du coefficient de conductivité thermique du matériau de la couche d'encrassement [W.m$^{-1}$.K$^{-1}$] ;
- l$n$ est un logarithme Népérien ; et
- e est l'épaisseur de la couche d'encrassement [m].

Lorsque le réacteur (R) n'est pas encrassé, l'épaisseur (e) de la couche d'encrassement est nulle et la différence de température ($\Delta\theta = \theta_w$

- $\theta_b$) peut être calculée selon la formule (Eq.6) suivante :

$$\Delta\theta = (\theta_w - \theta_b) = \frac{P}{2 \cdot \pi \cdot r \cdot L} \cdot \left(\frac{1}{h}\right) \quad \textbf{(Eq.6)},$$

dans laquelle les paramètres P, r, L et h ont la même signification que ci-dessus.

Dans le mode de réalisation de la sonde active dans lequel le générateur de chaleur (1) est plan, c'est à dire de forme parallélépipédique pour lequel chacune des 2 faces participe au transfert de chaleur de manière identique, on applique, pour le calcul de l'épaisseur (e) de la couche d'encrassement, la formule (Eq.9) suivante résultant des équations (Eq.7) et (Eq. 8):

$$P = K \cdot 2 \cdot L \cdot w \cdot (\theta_w - \theta_b) \,(\text{Eq.7})$$

avec

$$\frac{1}{K} = \frac{1}{h} + \frac{e}{\lambda_d} \cdot L \cdot w \quad (\text{Eq.8})$$

$$\boxed{\Delta\theta = \theta_w - \theta_b = \frac{P}{2.L \cdot w} \cdot \left(\frac{1}{h} + \frac{e}{\lambda_d} \cdot L \cdot w\right)} \quad (\text{Eq.9})$$

dans laquelle :

- P est la puissance thermique délivrée par le générateur de chaleur (1), la puissance P étant elle-même calculée à partir des grandeurs électriques (I, U), selon la formule (Eq.10) suivante :

$$P = I.U \,(\text{Eq.10})$$

- I est l'intensité imposée [A],
- U est la tension résultante (V)
- K est le coefficient global d'échange [W.m$^{-2}$.K$^{-1}$] ;
- L est la longueur du générateur de chaleur (1) [m] ;
- w est la largeur du générateur de chaleur (1) [m] ;
- $\theta_w$ est la température de la surface externe de la paroi du générateur de chaleur (1) [K] ;
- $\theta_b$ est la température du fluide circulant dans le réacteur (R) [K] ;
- h est la valeur de transfert de chaleur par convection [W.m$^{-2}$.K$^{-1}$] ; et
- $\lambda_d$ est la valeur du coefficient de conductivité thermique du matériau de la couche d'encrassement [W.m$^{-1}$.K$^{-1}$] ;
  Lorsque le réacteur (R) n'est pas encrassé, l'épaisseur (e) de la couche d'encrassement est nulle et la différence de température ($\Delta\theta = \theta_w$
- $\theta_b$) peut être calculée selon la formule (Eq.11) suivante :

$$(\theta_w - \theta_b) = \frac{P}{2 \cdot L \cdot w} \cdot \left(\frac{1}{h}\right) \quad \to O^+ (Eq.11)$$

L'épaisseur (e) de la couche d'encrassement est calculée selon la formule (Eq.7) suivante :

$$\Delta\theta = \theta_w - \theta_b = \frac{P}{2 \cdot L.w} \cdot \left(\frac{1}{h} + \frac{e}{\lambda_d} \cdot L.w\right)$$

ce qui équivaut à :

$$\Delta\theta = \frac{P}{L \cdot w} \cdot \frac{1}{h} + \frac{P}{2.L.w} \cdot \frac{e}{\lambda_d} \cdot L.w$$

En pratique, la différence de température $(\theta_w - \theta_b)_b$ tend vers $O^+$ pour des conditions propres et l'équation (10) peut se simplifier.
d'où l'expression de l'épaisseur de dépôt suivant (Eq.12) :

$$\Delta\theta \cong \frac{P}{2} \cdot \frac{e}{\lambda_d} \text{ soit } e \cong \Delta\theta \cdot \frac{2.\lambda_d}{P} \text{ (Eq. 12)}$$

En conséquence, il existe une proportionnalité directe entre la différence de température et l'épaisseur de dépôt.
L'invention a également pour objet un système pour la mesure de l'encrassement d'un réacteur (R) de chambrage inclus dans un dispositif pour la mise en oeuvre d'un procédé en continu, ledit système comprenant :

(i) localisée dans le coeur de l'écoulement fluidique du réacteur (R), une sonde passive constituée d'un générateur de chaleur (1), cylindrique ou plan, sur la surface externe duquel est fixée une première sonde thermique (2) ; et
(ii) localisée à proximité du générateur de chaleur (1), une sonde passive constituée d'une deuxième sonde thermique (3) ;

Les caractéristiques des différents moyens inclus dans le système de mesure ci-dessus ont été décrits précédemment dans la présente description. Certaines de ces caractéristiques sont brièvement rappelées ci-dessous.
De préférence, les extrémités amont de la sonde active et de la sonde passive sont localisées, l'une par rapport à l'autre, dans le sens axial du réacteur (R), à une distance (l) de quelques centimètres et en accord avec l'encombrement des sondes (environnement proche). Afin de permettre une mesure précise de la valeur $\Delta\theta$, les extrémités amont, par rapport au sens de l'écoulement fluidique dans le réacteur (R), de la sonde active et de la sonde passive sont de préférence localisées, l'une par rapport à l'autre, dans le sens axial du réacteur (R), à une distance (l) d'au plus 20 cm, mieux d'au plus 10 cm, et encore mieux d'au plus 5 cm.
Préférentiellement, la sonde active et la sonde passive sont localisées, l'une par rapport à l'autre, dans le sens radial du réacteur (R), à une distance (r) d'au plus 0,5 . R, R étant le rayon du réacteur (R).
De manière préférée, la sonde active et la sonde passive sont localisées sur la même section transversale du réacteur (R).
Avantageusement, le générateur de chaleur consiste en une sonde thermique à fil chaud, de préférence à fil de platine.
La sonde (2) est un thermocouple fin de manière à avoir un encombrement stérique négligeable sur la sonde active (1) et un temps de réponse rapide (faible inertie thermique).
La sonde (3) peut être indifféremment un thermocouple ou une sonde de platine (Pt 100), les critères de choix étant effectués selon le degré de mesure désiré à la faible inertie thermique.
En référence à la figure 1, dans un mode de réalisation particulier du système de mesure de l'invention, la sonde active et la sonde passive sont reliées à un moyen (4) de traitement des signaux, de préférence un moyen de traitement numérique des signaux, tel qu'un ordinateur.
Avantageusement, le moyen (4) comprend des moyens de réception des signaux qui sont fournis respectivement par la sonde active et la sonde passive, et plus particulièrement par (i) la première sonde thermique (2) incluse dans la sonde active et (ii) la deuxième sonde thermique (3). Les signaux fournis par les sondes (2 ; 3) sont reçus par le moyen (4), soit directement, soit indirectement. Dans un mode de réalisation particulier du système de l'invention, les signaux fournis par les sondes thermiques (2 ; 3) sont reçus directement par une interface (5) qui convertit les signaux analogiques, tout particulièrement les valeurs d'intensité de courant, fournis par les sondes thermiques (2 ; 3) en des signaux numériques de valeur des températures ($\theta_w$ ; $\theta_b$) qui sont ensuite transmis vers le moyen (4) de traitement des signaux.

L'interface (5) est aussi reliée au générateur de chaleur (1). L'interface (5) reçoit du générateur de chaleur (1) les signaux de commande qui permettent de générer une quantité de chaleur contrôlée dans la sonde active. Lorsque le générateur de chaleur (1) consiste en une sonde thermique à fil chaud, comme décrit précédemment, l'interface (5) fournit des signaux de commande de valeur de différence de potentiel (U) et d'intensité de courant (I) qui sont appliquées audit générateur de chaleur grâce à un générateur de courant continu (6). Dans certains modes de réalisation du système de mesure de l'invention, comme décrit dans la figure 1, le générateur de chaleur (1), qui consiste en une sonde thermique à fil chaud, est aussi relié à un générateur (6) de courant continu. Dans ce cas particulier, le générateur de courant électrique (6) délivre au générateur de chaleur (1) une valeur de différence de potentiel (U) et une valeur d'intensité de courant (I) qui sont fixées par l' l'opérateur. Les valeurs de différence de potentiel (U) et de courant (I) sont maintenues constantes pendant toute la durée de mise en oeuvre de la méthode de mesure de l'encrassement du réacteur (R) selon l'invention. Au début de la méthode, les valeurs de différence de potentiel (U) et de courant (I) qui sont délivrées au générateur de chaleur par l'intermédiaire du générateur de courant électrique (6) sont fixées par l'opérateur , de manière à ce que la valeur de température ($\theta_w$) de la surface externe de la paroi du générateur de chaleur (1) soit identique, ou quasi-identique, à la valeur de température ($\theta_b$) du fluide en circulation dans le réacteur (R), lorsque le réacteur (R) n'est pas encrassé.

Pendant toute la durée d'un procédé de traitement d'un fluide dans lequel on utilise le réacteur (R), l'interface (5) reçoit les signaux de mesure des températures ($\theta_w$) et ($\theta_b$) qui sont fournis par les sondes thermiques (2 ; 3). L'interface (5) est reliée au moyen (4) de traitement de signal. Les différents signaux analogiques envoyés par la sonde active et la sonde passive sont convertis en des signaux numériques par l'interface (5). Puis les signaux numériques générés par l'interface (5) sont transmis au moyen (4) de traitement du signal.

Le moyen (4) de traitement du signal comprend, notamment, un moyen de calcul de la valeur ($\Delta\theta = \theta_w - \theta_b$) pour chaque instant de mise en oeuvre du système de mesure de l'invention. Préférentiellement, le moyen (4) de traitement du signal comprend au moins un moyen qui génère une représentation graphique de la variation de la valeur ($\Delta\theta = \theta_w - \theta_b$) en fonction du temps, qui illustre l'évolution de l'état d'encrassement du réacteur (R), tel qu'un écran d'ordinateur ou un dispositif d'impression.

En général, le moyen (4) de traitement du signal consiste en un ordinateur numérique comprenant un écran et, le cas échéant, une imprimante. Le moyen (4) comprend au moins une mémoire qui est chargée avec un programme d'ordinateur comprenant une suite d'instructions permettant le calcul, à partir des données de mesures qui lui sont transmises par l'interface (5), des différents paramètres pertinents permettant à l'utilisateur de déterminer l'état d'encrassement du réacteur (R) à un instant donné, notamment le calcul du paramètre ($\Delta\theta = \theta_w - \theta_b$) et l'estimation de l'épaisseur de dépôt (e) si la conductivité thermique de ce dernier est connue

L'invention a également pour objet un dispositif pour la mise en oeuvre d'un procédé en continu de traitement d'un fluide, caractérisé en ce qu'au moins l'un des réacteurs inclus dans ledit dispositif est équipé d'un système de mesure de l'encrassement tel que défini ci-dessus.

La présente invention est en outre illustrée, sans pour autant y être limitée, aux exemples qui suivent.

## EXEMPLES

### Exemple 1 : Construction d'un système de mesure de l'encrassement selon l'invention.

1. Pour réaliser la sonde active, on utilise :

- une sonde thermique (1) à fil de platine gaînée Inox (L= 125 mm, Lactive = 30 mm, Ø = 1.6 mm) du type D52-1716-X-Cu2-125-MMCU commercialisée par la Société CORAM (France).
- une sonde thermique (2) à thermocouple de type K, du type DC2-Ko25L-1000i; L = 1000), Ø=0,25 mm) commercialisée par la Société CORAM (France)

La sonde thermique (2) à thermocouple est fixée par brasure longitudinalement sur la sonde thermique (1) à fil de platine. La sonde thermique (2) est localisée à la moitié de la longueur de la zone active de la sonde thermique (1) à fil de platine.

**2.** Pour réaliser la sonde passive, on utilise une sonde thermique (3) à fil de platine du type UE 902327, Class B (L = 25 mm, Ø=3 mm) commercialisée par la Société Heraeus (Suède).

**3.** Les deux pôles de la sonde thermique (1) à fil de platine sont électriquement reliés à un générateur de courant continu (6) ajustable délivrant un courant allant de 0 à 50 mA.

**4.** De plus, les deux pôles de la sonde thermique (1) à fil de platine sont électriquement reliés à une interface (5) qui est une carte de conversion de signaux analogique/numérique du type module 6B, commercialisée par la Société Analog Devices Inc. (Etats-Unis d'Amérique).

**5.** Les sondes thermiques à thermocouple (2 ; 3) sont électriquement reliées à l'interface (5).

**6.** L'interface (5) est elle-même reliée, par l'intermédiaire d'une connexion normalisée de type RS232, à un ordinateur du type ordinateur personnel (PC) dont la mémoire a été chargée avec un programme d'ordinateur comprenant les instructions de calcul adéquates.

**7.** Le capteur est placé dans un réacteur (R) de chambrage du type conduite cylindrique (Ø = 25mm, acier inoxydable 55316 L). La sonde active est disposée dans le réacteur (R) de manière à ce que son axe longitudinal soit parallèle à l'axe longitudinal du réacteur. La sonde active est fixée approximativement au centre de la lumière interne du réacteur (R).

La sonde passive est fixée dans le réacteur (R) de chambrage, à proximité de la sonde active et est donc éloignée des parois du réacteur.

**Exemple 2 : Mise en oeuvre du système de mesure de l'encrassement d'un réacteur (R) selon l'invention.**

**1. Protocole expérimental**

Le cas retenu concerne les phénomènes d'encrassement dans les zones de chambrage pour lesquels peu d'information sont recherchées. Le chambrage reste cependant une zone extrêmement importante pour le produit (cuisson, maturation) induisant tout autant, voir plus que le chauffage des phénomènes de déposition (encrassement).

L'installation utilisée se compose des éléments suivants placés en série : une cuve de lancement (eau) et d'une cuve de produit (A, B et C), et une vanne 3 voies permettant d'alimenter le circuit en eau ou en produit, d'une pompe volumétrique, d'une zone de chauffage (échangeur de chaleur à plaque, ou échangeur tubulaire) d'une zone de chambrage (tube cylindrique, Ø =25mm), d'une zone de refroidissement (échangeur de chaleur tubulaire) et d'une cuve de réception.

L'installation est alimentée avec de l'eau (stabilisation des paramètres thermiques et hydrauliques), puis, à) partir du temps T = 0 minute, avec les produits A, B et C. Les produits A, B et C sont des produits de type laitier avec des conditions physico-chimiques modifiées pour générer des phénomènes d'encrassement plus ou moins prononcé.

Le système de mesure d'encrassement selon l'invention a été placé dans le réacteur de chambrage localisé en aval, par rapport au sens de circulation du fluide, du réacteur échangeur de chaleur, respectivement aux températures de 95°C et 98°C.

**2. Résultats**

On a suivi la variation de la différence de température ($\Delta\theta = \theta_w - \theta_b$) comme le paramètre d'état d'encrassement du réacteur de chambrage, au cours du temps de fonctionnement de l'installation de traitement de produits laitiers, puis connaissant la conductivité thermique du dépôt formé ($\lambda_d$ =0.27 W.m$^{-1}$.K$^{-1}$) il a été possible d'estimer l'épaisseur du dépôt.

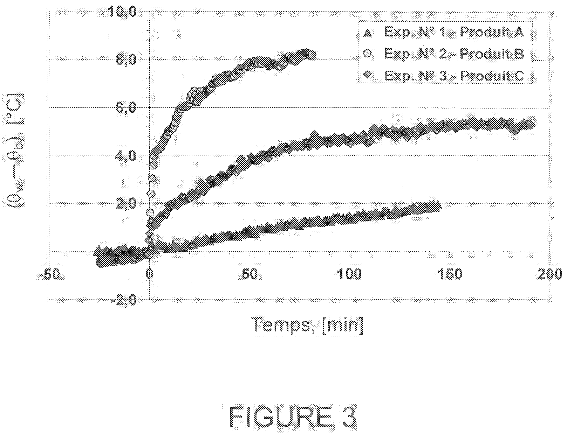

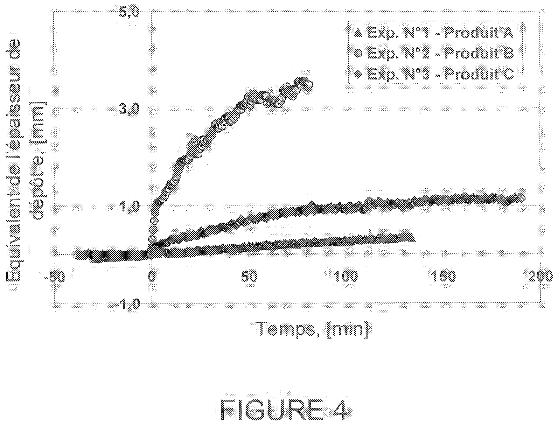

Les résultats sont présentés sur les Figures 3 et 4, dans laquelle, pour chacune des conditions opératoires de température ci-dessus, on a réalisé un suivi de l'état de l'encrassement du réacteur de chambrage au cours du temps. Sur la figure 3, l'état d'encrassement du réacteur de chambrage est mesuré comme la valeur de la différence de température ($\Delta\theta = \theta_w - \theta_b$). Sur la figure 4, l'état d'encrassement du réacteur de chambrage est mesuré grâce à l'estimation de l'épaisseur de dépôt, e.

L'encrassement varie en cinétique et en intensité selon les types de traitement et de produit, ce qui est illustré dans la figure 4.

Les différents essais ont été réalisés dans les conditions opératoires précisées dans le tableau 1.

**Tableau 1 : Conditions opératoires**

| N° essai | Produit | Débit L/h | Température d'entrée,°C | Température sortie et chambrage, °C | Type de Chauffage | Type de Chambrage | Durée (min) |
|---|---|---|---|---|---|---|---|
| 1 | A | 200 | 16 | 95 | Echangeurs de Chaleur à plaques | Tube 1" Ø=25mm et Tube 1"1/2 Ø=36mm | 1150 |
| 2 | B | 300 | 4 | 98 | Echangeurs de Chaleur tubulaire | Tube 1" | 880 |
| 3 | C | 300 | 4 | 98 | Echangeurs de Chaleur tubulaire | Tube 1" | 2200 |

**Revendications**

1. Méthode pour la mesure de l'encrassement d'un réacteur (R), ledit réacteur étant un organe d'une installation de traitement d'un fluide dans lequel circule un fluide et qui comprend un système de mesure de l'encrassement comprenant :

   (i) localisée dans le coeur de l'écoulement fluidique du réacteur (R), une sonde active constituée d'un générateur de chaleur (1), cylindrique ou plan, sur la surface externe duquel est fixée une première sonde thermique (2) ; et (ii) localisée à proximité du générateur de chaleur (1), également dans le coeur de l'écoulement fluidique du réacteur (R) une sonde passive constituée d'une deuxième sonde thermique (3) ; et

   ladite méthode comprenant les étapes suivantes :

   a) porter la surface externe de la paroi dudit générateur de chaleur (1) à une première température ($\theta_w$), la Première température ($\theta_w$) étant mesurée avec la première sonde thermique (2), et différant d'au plus de +0,5°C par rapport à une deuxième température ($\theta_b$) du fluide en circulation dans le réacteur (R) en l'absence d'encrassement, la deuxième température ($\theta_b$) étant mesurée avec la seconde sonde thermique (3) ;
   b) mesurer, au cours du temps de fonctionnement du réacteur (R), respectivement les températures ($\theta_w$) et ($\theta_b$) et calculer la valeur de différence $\Delta\theta$ ($\Delta\theta = \theta_w - \theta_b$) ; et
   c) déterminer l'état d'encrassement du réacteur (R), qui varie avec la valeur de $\Delta\theta$.

2. Méthode selon la revendication 1, **caractérisée en ce que** les extrémités amont de la sonde active et de la sonde passive sont localisées, l'une par rapport à l'autre, dans le sens axial du réacteur (R), à une distance (l) de quelques cm et en accord avec l'encombrement des sondes (environnement proche).

3. Méthode selon la revendication 1, **caractérisée en ce que** la sonde active et la sonde passive sont localisées sur la même section transversale du réacteur (R).

4. Méthode selon l'une quelconque des revendications 1 à 3, **caractérisée en ce que** la sonde active et la sonde passive sont localisées, l'une par rapport à l'autre, dans le sens radial du réacteur (R), à une distance (r) d'au plus 0,5 R, R étant le rayon du réacteur (R).

5. Méthode selon l'une des revendications 1 à 4, **caractérisée en ce que** la première sonde thermique (2) consiste en une sonde à thermocouple et **en ce que** la seconde sonde thermique (3) est (i) soit une sonde à thermocouple, (ii) soit une sonde de platine.

6. Méthode selon l'une quelconque des revendications 1 à 5, **caractérisée en ce que** le générateur de chaleur (1) consiste en une sonde thermique à fil chaud.

7. Méthode selon la revendication 6, **caractérisée en ce que** le générateur de chaleur (1) consiste en une sonde thermique à fil de platine.

**8.** Méthode selon l'une des revendications 6 ou 7, **caractérisée en ce que** le générateur de chaleur (1) consiste en un générateur cylindrique, l'épaisseur (e) de la couche d'encrassement étant calculée selon la formule suivante :

$$e \cong r.\left( Exp\left( \Delta\theta . \frac{2\pi L}{P} . \lambda_d \right) - 1 \right) \ (Eq.5)$$

avec $\Delta\theta = (\theta_w - \theta_b)$
dans laquelle :

- $\Delta\theta$ est la différence des températures $(\theta_w - \theta_b)$ [K] ;
- L est la longueur du générateur de chaleur (1) [m] ;
- P est la puissance dissipée dans le fil chaud [W] ;
- r est le rayon du générateur de chaleur (1) [m] ;
- $\lambda_d$ est la valeur du coefficient de conductivité thermique du matériau de la couche d'encrassement [W.m$^{-1}$.K$^{-1}$] ; et
- e est l'épaisseur de la couche d'encrassement [m].

**9.** Méthode selon l'une des revendications 6 ou 7, **caractérisée en ce que** le générateur de chaleur (1) consiste en un générateur plan, l'épaisseur (e) de la couche d'encrassement étant calculée selon la formule suivante :

$$\Delta\theta \cong \frac{P}{2} . \frac{e}{\lambda_d} \text{ soit } e \cong \Delta\theta . \frac{2.\lambda_d}{P},$$

avec :

- $\Delta\theta$ est la différence des températures $(\theta_w - \theta_b)$ [K] ;
- $\lambda_d$ est la valeur du coefficient de conductivité thermique du matériau de la couche d'encrassement [W.m$^{-1}$.K$^{-1}$] ; et
- P est la puissance thermique délivrée par le générateur de chaleur (1) [W].

**10.** Méthode selon l'une des revendications 1 à 9, **caractérisée en ce que** le réacteur (R) est inclus dans un dispositif pour la mise en oeuvre d'un procédé en continu de traitement d'un fluide.

**11.** Méthode selon l'une des revendications 1 à 10, **caractérisée en ce que** le réacteur (R) consiste en un réacteur de type échangeur de chaleur.

**12.** Méthode selon l'une des revendications 1 à 10, **caractérisée en ce que** le réacteur (R) consiste en un réacteur de chambrage.

**13.** Méthode selon l'une des revendications 1 à 12, **caractérisée en ce qu'**elle est mise en oeuvre pour la mesure de l'encrassement d'un réacteur (R) dans un procédé en continu de fabrication d'un produit agro-alimentaire.

**14.** Système pour la mesure de l'encrassement d'un réacteur (R), inclus dans un dispositif pour la mise en oeuvre d'un procédé en continu, selon la méthode telle que définie dans l'une des revendications 1 à 13, **caractérisé en ce que** ledit système comprend :

(i) une sonde active destinée à être positionnée dans le coeur de l'écoulement fluidique du réacteur (R), ladite sonde active étant constituée

(a) d'un générateur de chaleur (1), cylindrique ou plan, et
(b) d'une première sonde thermique (2) fixée sur la surface externe dudit générateur de chaleur (1),

(ii) une sonde passive destinée à être positionnée dans le coeur de l'écoulement fluidique du réacteur (R), constituée d'une deuxième sonde thermique (3),
(iii) un moyen (4) de traitement de signaux relié, par l'intermédiaire d'une interface (5), audit générateur de chaleur (1), à ladite première sonde thermique (2) et à ladite seconde sonde thermique (3),

laquelle interface (5) reçoit, du générateur de chaleur (1), les signaux de commande pour générer une quantité de chaleur contrôlée dans ladite sonde active de manière à porter la surface externe de la paroi dudit générateur de chaleur (1) à une première température ($\theta_w$), ladite première température ($\theta_w$) étant mesurée avec ladite première sonde thermique (2), et ladite première température ($\theta_w$) différant d'au plus de +0,5°C par rapport à une seconde température ($\theta_b$) du fluide en circulation dans le réacteur (R) en l'absence d'encrassement, ladite seconde température ($\theta_b$) étant mesurée avec ladite seconde sonde thermique (3),
lequel moyen de traitement (4) comprend un moyen de comparaison de la valeur de la première température ($\theta_w$) fournie par ladite première sonde thermique (2) et de la valeur de la seconde température ($\theta_b$) fournie par ladite seconde sonde thermique (3), pour la fourniture de la valeur de différence ($\Delta\theta = \theta_w - \theta_b$).

15. Système de mesure selon la revendication 14, **caractérisé en ce que** le générateur de chaleur (1) consiste en une sonde thermique à fil chaud.

16. Système de mesure selon la revendication 15, **caractérisé en ce que** le générateur de chaleur consiste en une sonde thermique à fil de platine.

17. Système selon l'une des revendications 14 à 16, **caractérisé en ce que** la première sonde thermique (2), consiste en une sonde à thermocouple et **en ce que** la seconde sonde thermique (3) est (i) soit une sonde à thermocouple, (ii) soit une sonde de platine.

18. Dispositif pour la mise en oeuvre d'un procédé en continu, **caractérisé en ce qu'**au moins l'un des réacteurs inclus dans ledit dispositif est équipé d'un système de mesure de l'encrassement selon l'une des revendications 14 à 17.

19. Procédé d'analyse des caractéristiques d'un réacteur (R) vis-à-vis de la formation d'un dépôt d'encrassement **caractérisé en ce qu'**il comprend les étapes suivantes :

a) placer un système pour la mesure de l'encrassement selon l'une quelconque des revendications 14 à 18 dans un réacteur (R) à tester non encrassé, dans lequel circule un fluide test ;
b) appliquer, au générateur de chaleur (1) dudit système, une valeur d'intensité de courant ($I_1$) pour laquelle ledit système fonctionne comme détecteur d'encrassement, et mesurer la valeur de ($\Delta\theta$) définie dans la revendication 1;
c) appliquer, au générateur de chaleur (1) dudit système, une valeur d'intensité de courant ($I_2$), ladite valeur d'intensité ($I_2$) étant

- supérieure à ($I_1$) de l'étape b), valeur pour laquelle le générateur (1) provoque un échauffement des parois dudit système de mesure susceptible d'induire un encrassement desdites parois, ou
- inférieure à ($I_1$) de l'étape b), valeur pour laquelle le générateur (1) est utilisé comme sonde de température;

d) appliquer, au générateur de chaleur (1) dudit système, la valeur d'intensité de courant ($I_1$) de l'étape b), et mesurer la valeur de ($\Delta\theta$);
e) réitérer les étapes c) et d) pendant le nombre de cycles désiré ; et
f) déterminer les caractéristiques du réacteur (R) vis-à-vis de la formation d'un dépôt d'encrassement par comparaison de la valeur ($\Delta\theta$) déterminée à l'étape b) avec les valeurs ($\Delta\theta$) déterminées aux étapes d).

## Patentansprüche

1. Verfahren zur Messung der Verschmutzung eines Reaktors (R), wobei der Reaktor ein Element einer Anlage zur Bearbeitung eines Fluids ist, in der ein Fluid zirkuliert und die ein System zum Messen der Verschmutzung umfasst, umfassend:

(i) eine im Kern des Fluidabflusses aus dem Reaktor (R) angeordnete Aktivsonde, die von einem zylindrischen oder flachen Wärmegenerator (1) gebildet ist, auf dessen Außenfläche eine erste Wärmesonde (2) befestigt ist; und
(ii) eine in der Nähe des Wärmegenerators (1), auch im Kern des Fluidabflusses aus dem Reaktor (R), angeordnete, eine Passivsonde, die von einer zweiten Wärmesonde (3) gebildet ist; und

wobei das Verfahren die folgenden Schritte umfasst:

a) Bringen der Außenfläche der Wand des Wärmegenerators (1) auf eine erste Temperatur ($\theta_w$), wobei die erste Temperatur ($\theta_w$) mit der ersten Wärmesonde (2) gemessen wird und sich um mehr als +0,5°C von einer zweiten Temperatur ($\theta_b$) des im Reaktor (R) zirkulierenden Fluids ohne Verschmutzung unterscheidet, wobei die zweite Temperatur ($\theta_b$) mit der zweiten Wärmesonde (3) gemessen wird;
b) während der Betriebszeit des Reaktors (R) Messen der Temperaturen ($\theta_w$) bzw. ($\theta_b$) und Berechnen des Differenzwerts $\Delta\theta$ ($\Delta\theta = \theta_w - \theta_b$); und
c) Bestimmen des Verschmutzungszustands des Reaktors (R), der mit dem Wert von $\Delta\theta$ variiert.

2. Verfahren nach Anspruch 1, **dadurch gekennzeichnet, dass** die stromaufwärtigen Enden der Aktivsonde und der Passivsonde in Bezug zueinander in Axialrichtung des Reaktors (R) in einem Abstand (1) von einigen cm und in Übereinstimmung mit dem Platzbedarf der Sonden (nahe Umgebung) angeordnet sind.

3. Verfahren nach Anspruch 1, **dadurch gekennzeichnet, dass** die Aktivsonde und die Passivsonde auf demselben Querschnitt des Reaktors (R) angeordnet sind.

4. Verfahren nach einem der Ansprüche 1 bis 3, **dadurch gekennzeichnet, dass** die Aktivsonde und die Passivsonde in Bezug zueinander in Radialrichtung des Reaktors (R) in einem Abstand (r) von höchstens 0,5 R, angeordnet sind, wobei R der Radius des Reaktors (R) ist.

5. Verfahren nach einem der Ansprüche 1 bis 4, **dadurch gekennzeichnet, dass** die erste Wärmesonde (2) aus einer Sonde mit Thermoelement besteht, und dass die zweite Wärmesonde (3) entweder (i) eine Sonde mit Thermoelement oder (ii) eine Platinsonde ist.

6. Verfahren nach einem der Ansprüche 1 bis 5, **dadurch gekennzeichnet, dass** der Wärmegenerator (1) aus einer Heißdraht-Wärmesonde besteht.

7. Verfahren nach Anspruch 6, **dadurch gekennzeichnet, dass** der Wärmegenerator (1) aus einer Platindraht-Wärmesonde besteht.

8. Verfahren nach einem der Ansprüche 6 oder 7, **dadurch gekennzeichnet, dass** der Wärmegenerator (1) aus einem zylindrischen Generator besteht, wobei die Dicke (e) der Verschmutzungsschicht nach folgender Formel berechnet wird:

$$e \cong r\left( Exp\left( \Delta\theta \cdot \frac{2\pi\,L}{P} \cdot \lambda_d \right) - 1 \right) \quad \text{(Formel 5)}$$

wobei $\Delta\theta = (\theta_w - \theta_b)$
wobei:

- $\Delta\theta$ die Differenz der Temperaturen ($\theta_w - \theta_b$) ist [K] ;
- L die Länge des Wärmegenerators (1) ist [m];
- P die in dem Heißdraht abgeleitete Leistung ist [W];
- r der Radius des Wärmegenerators (1) ist [m];
- $\lambda_d$ der Wert des Wärmeleitkoeffizienten des Materials der Verschmutzungsschicht ist [W.m$^{-1}$.K$^{-1}$]; und
- e die Dicke der Verschmutzungsschicht ist [m] .

9. Verfahren nach einem der Ansprüche 6 oder 7, **dadurch gekennzeichnet, dass** der Wärmegenerator (1) aus einem flachen Generator besteht, wobei die Dicke (e) der Verschmutzungsschicht nach folgender Formel berechnet wird:

$$\Delta\theta \cong \frac{P}{2} \cdot \frac{e}{\lambda_d}, \text{ also } \quad e \cong \Delta\theta \cdot \frac{2\lambda_d}{P},$$

wobei:

- $\Delta\theta$ die Differenz der Temperaturen ($\theta_w$ - $\theta_b$) ist [K];
- $\lambda_d$ der Wert des Wärmeleitkoeffizienten des Materials der Verschmutzungsschicht ist [W.m$^{-1}$.K$^{-1}$]; und
- P die vom Wärmegenerator (1) gelieferte Wärmeleistung ist [W].

10. Verfahren nach einem der Ansprüche 1 bis 9, **dadurch gekennzeichnet, dass** der Reaktor (R) in eine Vorrichtung für den Einsatz eines kontinuierlichen Fluidbehandlungsverfahrens eingeschlossen ist.

11. Verfahren nach einem der Ansprüche 1 bis 10, **dadurch gekennzeichnet, dass** der Reaktor (R) aus einem Reaktor vom Typ Wärmetauscher besteht.

12. Verfahren nach einem der Ansprüche 1 bis 10, **dadurch gekennzeichnet, dass** der Reaktor (R) aus einem Kammerreaktor besteht.

13. Verfahren nach einem der Ansprüche 1 bis 12, **dadurch gekennzeichnet, dass** es zum Messen der Verschmutzung eines Reaktors (R) in eines kontinuierlichen Vorgangs zur Herstellung eines Nahrungsmittels eingesetzt wird.

14. System zur Messung der Verschmutzung eines Reaktors (R), das in eine Vorrichtung für den Einsatz eines kontinuierlichen Vorgangs nach dem Verfahren, wie in einem der Ansprüche 1 bis 13 definiert, eingeschlossen ist, **dadurch gekennzeichnet, dass** das System umfasst:

(i) eine Aktivsonde, die dazu bestimmt ist, im Kern des Fluidabflusses aus dem Reaktor (R) angeordnet zu sein, wobei die Aktivsonde aus

(a) einem zylindrischen oder flachen Wärmegenerator (1), und
(b) einer ersten Wärmesonde (2), die auf der Außenfläche des Wärmegenerators (1) befestigt ist,

gebildet ist,
(ii) eine Passivsonde, die dazu bestimmt ist, im Kern des Fluidabflusses aus dem Reaktor (R) angeordnet zu sein, und aus einer zweiten Wärmesonde (3) gebildet ist,
(iii) ein Signalverarbeitungsmittel (4), das über eine Schnittstelle (5) mit dem Wärmegenerator (1), mit der ersten Wärmesonde (2) und mit der zweiten Wärmesonde (3) verbunden ist,

wobei die Schnittstelle (5) vom Wärmegenerator (1) die Steuersignale empfängt, um eine kontrollierte Wärmemenge in der Aktivsonde zu erzeugen, um die Außenfläche der Wand des Wärmegenerators (1) auf eine erste Temperatur ($\theta_w$) zu bringen, wobei die erste Temperatur ($\theta_w$) mit der ersten Wärmesonde (2) gemessen wird, und wobei sich die erste Temperatur ($\theta_w$) um höchstens +0,5°C von einer zweiten Temperatur ($\theta_b$) des im Reaktor (R) zirkulierenden Fluids ohne Verschmutzung unterscheidet, wobei die zweite Temperatur ($\theta_b$) mit der zweiten Wärmesonde (3) gemessen wird, wobei das Verarbeitungsmittel (4) ein Mittel zum Vergleichen des Werts der ersten Temperatur ($\theta_w$), der von der ersten Wärmesonde (2) geliefert wird, und des Werts der zweiten Temperatur ($\theta_b$), der von der zweiten Wärmesonde (3) geliefert wird, umfasst, um den Differenzwert ($\Delta\theta = \theta_w - \theta_b$) zu liefern.

15. Messsystem nach Anspruch 14, **dadurch gekennzeichnet, dass** der Wärmegenerator (1) aus einer Heißdraht-Wärmesonde besteht.

16. Messsystem nach Anspruch 15, **dadurch gekennzeichnet, dass** der Wärmegenerator aus einer Platindraht-Wärmesonde besteht.

17. System nach einem der Ansprüche 14 bis 16, **dadurch gekennzeichnet, dass** die erste Wärmesonde (2) aus einer Sonde mit Thermoelement besteht, und dass die zweite Wärmesonde (3) entweder (i) eine Sonde mit Thermoelement oder (ii) eine Platinsonde ist.

18. Vorrichtung für den Einsatz eines kontinuierlichen Vorgangs, **dadurch gekennzeichnet, dass** mindestens einer der Reaktoren, die in der Vorrichtung eingeschlossen sind, mit einem System zur Messung der Verschmutzung nach einem der Ansprüche 14 bis 17 ausgestattet ist.

19. Verfahren zur Analyse der Merkmale eines Reaktors (R) im Hinblick auf die Bildung einer Verschmutzungsablagerung, **dadurch gekennzeichnet, dass** es die folgenden Schritte umfasst:

a) Anordnen eines Systems zur Messung der Verschmutzung nach einem der Ansprüche 14 bis 18 in einem nicht verschmutzten zu testenden Reaktor (R), in dem ein Testfluid zirkuliert;

b) Anlegen, an den Wärmegenerator (1) des Systems, eines Stromstärkewerts ($I_1$), bei dem das System als Verschmutzungsdetektor funktioniert, und Messen des in Anspruch 1 definierten Werts ($\Delta\theta$);

c) Anlegen, an den Wärmegenerator (1) des Systems, eines Stromstärkewerts ($I_2$), wobei der Stromstärkewert ($I_2$)

- größer als ($I_1$) aus Schritt b) ist, ein Wert, bei dem der Generator (1) eine Erwärmung der Wände des Messsystems hervorruft, die geeignet ist, eine Verschmutzung der Wände zu induzieren, oder
- kleiner als ($I_1$) aus Schritt b) ist, ein Wert, bei dem der Generator (1) als Temperatursonde verwendet wird;

d) Anlegen, an den Wärmegenerator (1) des Systems, des Stromstärkewerts ($I_1$) aus Schritt b) und Messen des Werts ($\Delta\theta$);

e) Wiederholen der Schritte c) und d) während der gewünschten Anzahl von Zyklen; und

f) Bestimmen der Merkmale des Reaktors (R) im Hinblick auf die Bildung einer Verschmutzungsablagerung durch Vergleichen des in Schritt b) bestimmten Werts ($\Delta\theta$) mit den in Schritt d) bestimmten Werten ($\Delta\theta$).

**Claims**

1. A method for measuring fouling in a reactor (R), said reaction being an organ of an installation for the treatment of a fluid through which a fluid is flowing and which comprises a fouling measuring system comprising:

   (i) located into the core of the fluid flowing of the reactor (R), an active probe constituted of a heat generator (1), cylindrical or flat, onto which external surface a first temperature probe (2) is fastened; and
   (ii) located near to the heat generator (1), that is also within the core of the fluid flowing through the reactor (R), a passive probe consisting in a second temperature probe (3); and

   said method comprising the following steps of:

   a) warming up the wall external surface of said heat generator (1) to a first temperature ($\theta_w$), the first temperature ($\theta_w$) being measured by means of the first temperature probe (2) and differing at most by +0.5°C as compared to a second temperature ($\theta_b$) of the fluid flowing through the reactor (R) when there is no fouling, the second temperature ($\theta_b$) being measured by means of the second temperature probe (3);
   b) measuring, during the reactor (R) operating time, the temperatures ($\theta_w$) and ($\theta_b$), respectively, and calculating the difference value ($\Delta\theta = \theta_w - \theta_b$); and
   c) determining the fouling state of the reactor (R), which varies depending on the value of $\Delta\theta$.

2. A method according to claim 1, **characterized in that** the upstream ends of the active probe and of the passive probe are located, as opposed to one another, in the axial direction of the reactor (R), at a distance (I) of a few centimetres and according to the space requirement for the probes (close environment).

3. A method according to claim 1, **characterized in that** the active probe and the passive probe are located on the same cross section of the reactor (R).

4. A method according to any of the claims 1 to 3, **characterized in that** the active probe and the passive probe are located, as opposed to one another, in the radial direction of the reactor (R), at a distance (r) of at most 0.5 R, R being the radius of the reactor (R).

5. A method according to any of the claims 1 to 4, **characterized in that** the first temperature probe (2) consists in a thermocouple probe and **in that** the second temperature probe (3) is (i) either a thermocouple probe, (ii) or a platinum probe.

6. A method according to anyone of claims 1 to 5, **characterized in that** the heat generator (1) consists in a hot wire temperature probe.

7. A method according to claim 6, **characterized in that** the heat generator (1) consists in a platinum wire temperature probe.

8. A method according to any of the claims 6 or 7, **characterized in that** the heat generator (1) consists in a cylindrical generator, the thickness (e) of the fouling layer being calculated according to the following formula:

$$e \cong r \cdot \left( Exp\left( \Delta\theta \cdot \frac{2\pi L}{P} \cdot \lambda_d \right) - 1 \right) \quad (Eq.5)$$

with $\Delta0 = (\theta_w - \theta_b)$
wherein

- $\Delta\theta$ represents the temperature difference $(\theta_w - \theta_b)$ [K];
- L represents the length of the heat generator (1) [m];
- P represents the power dissipated in the hot wire [W];
- r represents the heat generator radius (1) [m];
- $\lambda_d$ represents the thermal conductivity coefficient value of the fouling layer material [W.m$^{-1}$.K$^{-1}$]; and
- e represents the fouling layer thickness [m].

9. A method according to anyone of claims 6 or 7, **characterized in that** the heat generator (1) consists in a flat generator, the thickness (e) of the fouling layer being calculated according to the following formula:

$$\Delta\theta \cong \frac{P}{2} \cdot \frac{e}{\lambda_d} \text{ soit } e \cong \Delta\theta \cdot \frac{2.\lambda_d}{P},$$

wherein:

- $\Delta\theta$ represents the temperature difference $(\theta_w - \theta_b)$ [K];
- $\lambda_d$ represents the thermal conductivity coefficient value of the fouling layer material [W.m$^{-1}$.K$^{-1}$]; and
- P represents the thermal power provided by the heat generator (1) [W].

10. A method according to any of claims 1 to 9, **characterized in that** the reactor (R) is included in a device for implementing a fluid treatment continuous process.

11. A method according to any of claims 1 to 10, **characterized in that** the reactor (R) consists in a reactor of the heat exchanger type.

12. A method according to any of claims 1 to 10, **characterized in that** the reactor (R) consists in a holding reactor.

13. A method according to any of claims 1 to 12, **characterized in that** it is implemented for measuring fouling in a reactor (R) in a continuous process for producing an agri-food product.

14. A fouling measuring system for a reactor (R) included in a device for implementing a continuous process, according to the process as specified in any of the claims 1 to 13, wherein the said system comprising:

(i) an active probe that is intended to be located within the core of the fluid flowing through the reactor (R), the said active probe being consisted in :

(a) a cylindrical or a flat heat generator (1), and
(b) a first temperature probe (2) which is fastened onto the external surface of the said heat generator (1) ;

(ii) a passive probe that is intended to be located within the core of the fluid flowing through the reactor (R), consisting in a second temperature probe (3),
(iii) signal processing means(4) connected, via an interface(5), to the heat generator (1), to the first and second temperature probes (2) and (3),

wherein said interface (5) receives, from the heat generator (1), the command signals to generate a controlled quantity of heat in the active probe in order to bring the wall external surface of said heat generator (1) to a first

temperature ($\theta_w$), the first temperature ($\theta_w$) being measured by means of the first temperature probe (2) and said first temperature ($\theta_w$) differing at most by +0.5°C as compared to a second temperature ($\theta_b$) of the fluid flowing through the reactor (R) when there is no fouling, the second temperature ($\theta_b$) being measured by means of the second temperature probe (3);

wherein signal processing means(4) comprises means to compare the value of the first temperature ($\theta_w$) provided by the first thermal probe (2) and the value of the second temperature ($\theta_b$) provided by the second thermal probe (3) to provide the difference value ($\Delta\theta = \theta_w - \theta_b$).

15. A measuring system according to claim 14, **characterized in that** the heat generator (1) consists in a hot wire temperature probe.

16. A measuring system according to claim 15, **characterized in that** the heat generator (1) consists in a platinum wire temperature probe.

17. A system according to any one of the claims 14 to 16, **characterized in that** the first temperature probe (2) consists in a thermocouple probe and **in that** the second temperature probe (3) is (i) either a thermocouple probe or (ii) a platinum probe.

18. A device for implementing a continuous process, **characterized in that** at least one of the reactors included in said device is provided with a fouling measuring system according to any of claims 14 to 17.

19. A method for analyzing the characteristics of a reactor (R) as regards the formation of a fouling deposit, comprising the following steps of:

a) setting a fouling measuring system as defined in anyone of claims 14 to 18 in a non fouled reactor (R) to be tested, through which a test fluid is flowing;
b) applying to the heat generator (1) of the said system, an amperage value ($I_1$) for which said device works as a fouling detector, and measuring the value of ($\Delta\theta$) as defined in claim 1;
c) applying to the heat generator (1) of said measuring system, an amperage value ($I_2$), said amperage value ($I_2$) being

- higher than ($I_1$) of step b), for which the generator (1) causes a warming up of the walls of said device which may induce fouling of said walls, or
- lower than ($I_1$) of step b), for which the generator (1) is used as a temperature probe;

d) applying to the heat generator (1) of said measuring system, an amperage value ($I_1$) of step b), and measuring the value of ($\Delta\theta$);
e) repeating the steps c) and d) for the desired number of cycles, and
f) determine the characteristics of the reactor (R) as regards the formation of a fouling deposit by comparing the value of ($\Delta\theta$) measured in step (b) with the values of ($\Delta\theta$) measured in steps d).

FIGURE 1

FIGURE 2

FIGURE 3

FIGURE 4

Figure 5

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0060633 A
- WO 0194876 A
- US 20020108911 A
- US 5590706 A
- EP 0155826 A
- US 5615733 A
- WO 0043762 A
- GB 2089512 A
- US 4383438 A

**Littérature non-brevet citée dans la description**

- **JOULIÉ R.** Mécanique des Fluides appliqués. 1998
- Single Phase convective heat transfer. **GNIDESKI V. ; CHURCHILL S.W.** Heat Exchanger Design Handbook. Hemisphere publishing corporation, 1983
- Heat Exchange Design Handbook. Hemisphere Publishing Corporation, 1983